# EUROPEAN PATENT APPLICATION

(11) **EP 1 541 149 A1**
(43) Date of publication of application: **15.06.2005**
(21) Application number: 03761814.7
(22) Date of filing: 26.06.2003
(51) Int. Cl.: A61K 31/47

(54) **PHOSPHODIESTERASE INHIBITOR**

(30) Priority: 26.06.2002 JP 2002185707
(71) Applicant: KYOWA HAKKO KOGYO CO., LTD., Chiyoda-ku, Tokyo 100-8185 (JP)
(72) Inventor: OSAKADA, Naoto c/o Pharmaceutical Research Inst., Nagaizumi-cho Sunto-gun, Shizuoka 411-87 (JP); HARUOKA, Motoko c/o Pharmaceutical Research Inst., Nagaizumi-cho Sunto-gun, Shizuoka 411-87 (JP); IKEDA, Ken c/o Pharmaceutical Research Inst., Nagaizumi-cho Sunto-gun, Shizuoka 411-87 (JP); TOKI, Shinichiro c/o Pharmaceutical Research Inst., Nagaizumi-cho Sunto-gun, Shizuoka 411-87 (JP); MIYAJI, Hiromasa c/o Pharmaceutical Research Inst., Nagaizumi-cho Sunto-gun, Shizuoka 411-87 (JP); SHIMADA, Junichi c/o Pharmaceutical Research Inst., Nagaizumi-cho Sunto-gun, Shizuoka 411-87 (JP)
(74) Representative: Casalonga, Axel
(86) International application number: PCT/JP2003/008079
(87) International publication number: WO 2004/002484

(57) **Abstract**

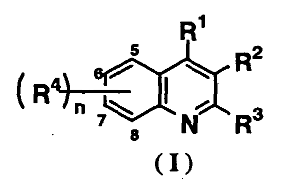

[wherein n represents an integer of from 1 to 4, R¹ represents substituted or unsubstituted lower alkyl, -C(=Y)R⁹ or the like, R² represents a hydrogen atom, substituted or unsubstituted lower alkyl or the like, R³ represents substituted or unsubstituted lower alkyl, substituted or unsubstituted aryl, a substituted or unsubstituted heterocyclic group or the like, and R⁴ represents halogen or the like.]

A phosphodiesterase 10A inhibitor which comprises a quinoline derivative represented by the above formula (I) or a pharmaceutically acceptable salt thereof as an active ingredient is provided.

Sequence Listing Free Text

<110> KYOWA HAKKO KOGYO CO., LTD

<120> PHOSPHODIESTERASE INHIBITERS

<130> 11494WO1

<140>
<141>

<150> JP 2002-185707
<151> 2002-06-26

<160> 4
<170> PatentIn Ver. 2.1

<210> 1
<211> 20
<212> DNA
<213> Artificial Sequence

<220>
<223> Description of Artificial Sequence: Synthetic DNA

<400> 1
ctgctgattg cgtgtctgtg                                                20

<210> 2
<211> 21
<212> DNA
<213> Artificial Sequence

<220>
<223> Description of Artificial Sequence: Synthetic DNA

<400> 2
caagatgagg atctgtaggt g                                              21

<210> 3
<211> 48
<212> DNA

<213> Artificial Sequence

<220>
<223> Description of Artificial Sequence: Synthetic DNA

<400> 3
gatcccgcca ccatggacta caaggacgat gacgacaaga gcaggtac                48

<210> 4
<211> 40
<212> DNA
<213> Artificial Sequence

<220>
<223> Description of Artificial Sequence: Synthetic DNA

<400> 4
ctgctcttgt cgtcatcgtc cttgtagtcc atggtggcgg                          40

## Description

### Technical Field

The present invention relates to a phosphodiesterase 10A (PDE10A) inhibitor which exhibits PDE10A inhibitory activity and comprises a quinoline derivative or a pharmaceutically acceptable salt thereof as an active ingredient which is useful for treating and/or preventing various diseases caused by enhancing the activity of PDE10A (for example, a neural disease such as Parkinson's disease, Huntington disease or Alzheimer disease, dyskinesia, hypogonadism, diabetes, an ischemic heart disease, hypertension, an inflammatory disease, a disease of the digestive system, an allergic disease, osteoporosis, pain, a malignant tumor or the like).

### Background Art

Cyclic nucleotides have been known to mediate cell responses to various extracellular stimuli such as a stimulus from G protein coupled receptors (GPCR). Phosphodiesterase (PDE) plays an important role in regulation of the intracellular concentration of these cyclic nucleotides by hydrolyzing cyclic nucleotides such as 5',3'-cyclic adenosine monophosphate (cAMP), 3',5'-cyclic guanosine monophosphate (cGMP) and the like [Pharmac. Ther., vol. 51, p. 13 (1991)].

In tissues of vertebrates, various PDE subtypes have been found, that hydrolyze cyclic nucleotides [Trend. Pharmacol. Sci. vol. 11, p. 150 (1990), Physiol. Rev. vol. 75, p. 725 (1995), Arch. Biochem. Biophys. vol. 322, p. 1 (1995), and Kidney International, vol. 55, p. 29 (1999)]. These have been to date classified into 11 families (PDE1~PDE11) according to biochemical characteristics, enzymological characteristics, homology of amino acid sequences by cloning of corresponding cDNAs, sensitivity to inhibitors and the like.

Regarding PDE10A which is one of the PDE subtypes, the expression of its mRNA has been identified in many tissues and organs such as striatum, testis, kidney, thyroid gland, pituitary gland, thalamus, cerebellum, heart, lungs and placenta, cells such as aortic smooth muscle cells and aortic endothelial cells, cells of cancers such as lung small cell carcinoma, breast cancer and large bowel cancer, and the like. Accordingly, the possibility that PDE10A is involved in diseases related to these cells, tissues and organs has been pointed out [J. Biol. Chem. vol. 274, p. 18438 (1999), Gene, vol. 234, p. 109 (1999), WO 01/29199 and Japanese Published Unexamined Patent Application No. 2001-161379].

From the view points of strong expression of mRNA of PDE10A and its enzymatic activity in the striatum, this enzyme is suggested to be involved, for example, onset or progression of Parkinson's disease, Huntington disease and the like, dyskinesia caused by long-term administration of L-DOPA (L-3,4-dihydroxyphenylalanine), and the like For example, it has been reported that in the striatum of model mice of Huntington disease, the expression of mRNA of PDE10A is different from that in the striatum of normal mice (WO 01/24781).

Further, PDE10A catalyzes hydrolysis from cGMP to GMP, and this hydrolase is present in the cavernous body. Accordingly, it is suggested that a PDE10A inhibitor possibly improves, for example, men's impotence and women's hypogonadism (Japanese Published Unexamined Patent Application No. 2000-23682).

In view of the foregoing, it is expected that an inhibitor having selectivity to PDE10A (PDE10A inhibitor) is useful for treating and/or preventing various diseases caused by enhancing the activity of PDE10A (for example, a neural disease such as Parkinson' s disease, Huntington disease or Alzheimer' s disease, dyskinesia, hypogonadism, diabetes, an ischemic heart disease, hypertension, an inflammatory disease, a disease of the digestive system, an allergic disease, osteoporosis, pain or a malignant tumor), and has the possibility as a treating agent with reduced side effects.

Meanwhile, it has been known that quinolinecarboxylic acid derivatives represented by general formula (C) 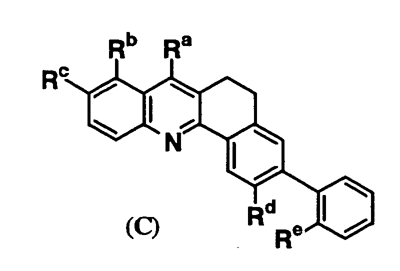 (wherein R^{a} represents carboxy or the like, R^{b} and R^{c}, which may be the same or different, each represent methyl, ethyl, a hydrogen atom, a fluorine atom or the like, and R^{d} and R^{e} each represent a hydrogen atom or R^{d} and R^{e} in combination represent a sulfur atom)
are useful as a cancer chemotherapeutic agent (Japanese Published Unexamined Patent Application No. 233661/90) and an immunosuppressor (WO 92/00739).

Tetracyclic quinolinecarboxylic acid derivatives (Japanese Published Unexamined Patent Application No. 231289/98) represented by general formula (D) 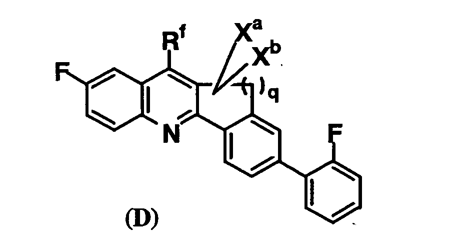 (wherein R^{f} represents carboxy or the like, X^{a} and X^{b}, which may be the same or different, each represent a hydrogen atom, lower alkyl or the like, and q represents an integer of from 1 to 4)
have been known, and it has been reported that the derivatives have activity of inhibiting antibody production in plaque-forming cells and an effect of preventing adjuvant arthritis (WO 93/22286).

Moreover, 4-quinolinecarboxylic acid derivatives represented by general formula (E) 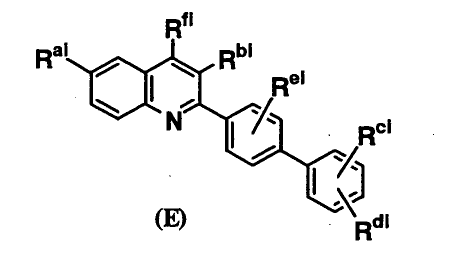 (wherein R^{ai} represents a fluorine atom or the like, R^{bi} represents methyl or the like, R^{ci}, R^{di} and R^{ei}, which may be the same or different, each represent halogen, a hydrogen atom or the like, and R^{fi} represents carboxy or its inorganic salt) have been known. The 4-quinolinecarboxylic acid derivatives have been reported as, for example, a dihydroorotic acid dehydrogenase inhibitor [WO 01/24785, Pharm. Res., vol. 15, p. 286 (1998) and Biochem. Pharmacol., vol. 40, p. 709 (1990) and vol. 56, p. 1053 (1998)], a potassium channel opener (Japanese Published Unexamined Patent Application No. 3144/96), an immunosuppressant [Japanese Published Unexamined Patent Application No. 313428/89, Bioorg. Med. Chem. Lett., vol. 5, p. 1549 (1995), WO 91/19498 and WO 97/42953], an antitumor agent (U.S. Patent No. 4680299, Japanese Published Unexamined Patent Application No. 121923/90 and Japanese Published Unexamined Patent Application No. 42367/85), an antiinflammatory agent [J. Rheumatol. , vol. 18, p. 855 (1991)], an antiviral agent [Antiviral. Res., vol. 20, p. 71 (1993) and WO 01/24785] and an agent for treating skin and mucosal epithelial diseases (Japanese Published Unexamined Patent Application No. 72163/90).

### Disclosure of the Invention

An object of the present invention is to provide a PDE10A inhibitor which exhibits PDE10A inhibitory activity and comprises a quinoline derivative or a pharmaceutically acceptable salt thereof as an active ingredient which is useful for treating and/or preventing various diseases caused by enhancing the activity of PDE10A function (for example, a neural disease such as Parkinson' s disease, Huntington disease or Alzheimer's disease, dyskinesia, hypogonadism, diabetes, an ischemic heart disease, hypertension, an inflammatory disease, a disease of the digestive system, an allergic disease, osteoporosis, pain, a malignant tumor or the like). Another object of the present invention is to provide a quinoline derivative or a pharmaceutically acceptable salt thereof which is useful for treating and/or preventing various diseases caused by enhancing the activity of PDE10A function.

The present invention relates to the following (1) to (33).
(1) A phosphodiesterase 10A (PDE10A) inhibitor which comprises a quinoline derivative represented by general formula (I) 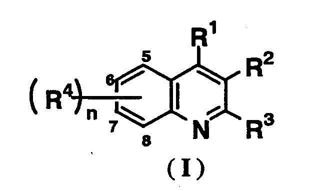 [wherein n represents an integer of from 1 to 4, R¹ represents substituted or unsubstituted lower alkyl, -C(=Y)R⁹ (wherein Y represents an oxygen atom or a sulfur atom, and R⁹ represents a hydrogen atom, hydroxy, substituted or unsubstituted lower alkyl, substituted or unsubstituted lower alkoxy, substituted or unsubstituted aryl, a substituted or unsubstituted heterocyclic group, amino, mono-lower alkylamino or di-lower alkylamino), hydroxy, halogen, cyano, amino, mono-lower alkylamino or di-lower alkyl amino, R² represents a hydrogen atom, amino, nitro, substituted or unsubstituted lower alkyl, substituted or unsubstituted lower alkoxy, -S(O)ₘR¹² (wherein R¹² represents substituted or unsubstituted lower alkyl or substituted or unsubstituted aryl, and m represents an integer of from 0 to 2), mono-lower alkylamino or di-lower alkylamino, R³ represents a hydrogen atom, halogen, hydroxy, substituted or unsubstituted lower alkyl, substituted or unsubstituted cycloalkyl, substituted or unsubstituted aryl or a substituted or unsubstituted heterocyclic group, or R² and R³ form a substituted or unsubstituted condensed ring together with two carbon atoms on roots thereof, and R⁴ represents a hydrogen atom, halogen, cyano, amino, nitro, substituted or unsubstituted lower alkyl, substituted or unsubstituted cycloalkyl, substituted or unsubstituted lower alkoxy, -S(O)ₘₐR^{12a} (wherein R^{12a} and ma have the same meanings as those of the above-mentioned R¹² and m respectively), -C(=Y¹)R^{9a} (wherein Y¹ and R^{9a} have the same meanings as those of the above-mentioned Y and R⁹ respectively), mono-lower alkylamino or di-lower alkylamino, and when n is an integer of 2 or more, R⁴s each may be the same or different],
   or a pharmaceutically acceptable salt thereof as an active ingredient.
(2) The PDE10A inhibitor according to above (1), wherein R¹ is substituted or unsubstituted lower alkyl, -C(=Y)R⁹ (wherein Y and R⁹ have the same meanings as those above-mentioned respectively), cyano or amino, and R² is substituted or unsubstituted lower alkyl.
(3) The PDE10A inhibitor according to above (1), wherein R¹ is methyl, hydroxymethyl, acetyl, carboxy, methoxycarbonyl, cyano or amino.
(4) The PDE10A inhibitor according to any one of above (1) to (3), wherein R³ is substituted or unsubstituted aryl or a substituted or unsubstituted heterocyclic group.
(5) The PDE10A inhibitor according to any one of above (1) to (3), wherein R³ is substituted or unsubstituted biphenylyl or substituted or unsubstituted piperazinyl.
(6) The PDE10A inhibitor according to any one of above (1) to (3), wherein R³ is substituted or unsubstituted biphenyl-4-yl or substituted or unsubstituted piperazin-1-yl.
(7) The PDE10A inhibitor according to any one of above (1) to (3), wherein R³ is general formula (A) 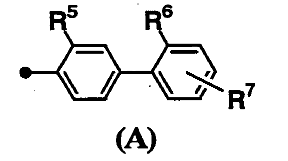 [wherein R⁵, R⁶ and R⁷, which may be the same or different, each represent a hydrogen atom, halogen, substituted or unsubstituted lower alkyl, substituted or unsubstituted lower alkoxy, aryl, substituted or unsubstituted lower alkanoyl or a substituted or unsubstituted heterocyclic group]
   or piperazin-1-yl having substituted or unsubstituted lower alkyl or substituted or unsubstituted aryl as a substituent on the 4-position.
(8) The PDE10A inhibitor according to any one of above (1) to (7), wherein n is 1, and R⁴ is halogen.
(9) A quinoline derivative represented by general formula (IA) 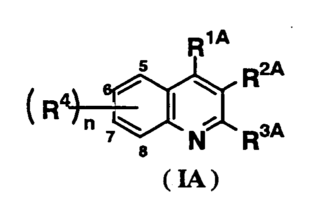 [wherein n and R⁴ have the same meanings as those above-mentioned respectively, R^{1A} represents lower alkyl, hydroxy lower alkyl, -C(=Y)R^{9A} (wherein Y has the same meaning as that above-mentioned, and R^{9A} represents a hydrogen atom, lower alkyl, lower alkoxy, amino, mono-lower alkylamino or di-lower alkylamino), cyano, amino, mono-lower alkylamino or di-lower alkylamino, R^{2A} represents amino, nitro, substituted or unsubstituted lower alkyl, substituted or unsubstituted lower alkoxy, -S(O)ₘR¹² (wherein R¹² and m have the same meanings as those above-mentioned respectively), mono-lower alkylamino or di-lower alkylamino, and R^{3A} represents a substituted or unsubstituted heterocyclic group or substituted or unsubstituted aryl, or R^{2A} and R^{3A} form cycloalkane condensed with a substituted or unsubstituted benzene ring together with two carbon atoms on roots thereof, provided that when R^{1A} is hydroxymethyl or -C(=O)R^{9B} (wherein R^{9B} represents a hydrogen atom, ethyloxy, n-propylamino or diethylamino), R^{3A} is not 4-cyclohexylphenyl, when R^{1A} is hydroxymethyl or -C(=O)R^{9C} (wherein R^{9C} represents methoxy, amino, mono-lower alkylamino or di-lower alkylamino) and R^{2A} is carboxyethyl or methoxycarbonylethyl, R^{3A} is not 4-(2-fluorophehyl)phenyl nor biphenyl-4-yl, and when R^{1A} is hydroxymethyl or -C(=O)R^{9D} (wherein R^{9D} represents amino or lower alkoxy) and R^{2A} is methyl, R^{3A} is not biphenyl-4-yl],
   or a pharmaceutically acceptable salt thereof.
(10) The quinoline derivative or the pharmaceutically acceptable salt thereof according to above (9), wherein R^{3A} is substituted or unsubstituted biphenylyl or substituted or unsubstituted piperazin-1-yl.
(11) The quinoline derivative or the pharmaceutically acceptable salt thereof according to above (9), wherein R^{3A} is substituted or unsubstituted biphenylyl or piperazin-1-yl having substituted or unsubstituted lower alkyl or substituted or unsubstituted aryl as a substituent on the 4-position.
(12) The quinoline derivative or the pharmaceutically acceptable salt thereof according to above (9), wherein R^{3A} is piperazin-1-yl having substituted or unsubstituted aryl as a substituent on the 4-position.
(13) The quinoline derivative or the pharmaceutically acceptable salt thereof according to any one of above (9) to (12), wherein R^{1A} is lower alkyl, hydroxy lower alkyl, -C(=O)R^{9E} (wherein R^{9A} represents lower alkyl or lower alkoxy) or cyano, and R^{2A} is substituted or unsubstituted lower alkyl.
(14) The quinoline derivative or the pharmaceutically acceptable salt thereof according to any one of above (9) to (13), wherein R^{1A} is methyl, hydroxymethyl, acetyl, methoxycarbonyl or cyano.
(15) The quinoline derivative or the pharmaceutically acceptable salt thereof according to any one of above (9) to (14), wherein n is 1, and R⁴ is halogen.
(16) A PDE10A inhibitor which comprises the quinoline derivative or the pharmaceutically acceptable salt thereof according to any one of above (9) to (15) as an active ingredient.
(17) An agent for treating and/or preventing a disease caused by enhancing the activity of PDE10A, which comprises the quinoline derivative or the pharmaceutically acceptable salt thereof according to any one of above (9) to (15) as an active ingredient.
(18) An agent for treating and/or preventing dyskinesia, which comprises the quinoline derivative or the pharmaceutically acceptable salt thereof according to any one of above (9) to (15) as an active ingredient.
(19) An antitumor agent which comprises the quinoline derivative or the pharmaceutically acceptable salt thereof according to any one of above (9) to (15) as an active ingredient.
(20) An agent for treating and/or preventing dyskinesia, which comprises a compound having PDE10A inhibitory activity or a pharmaceutically acceptable salt thereof as an active ingredient.
(21) A pharmaceutical composition which comprises the quinoline derivative or the pharmaceutically acceptable salt thereof according to any one of above (9) to (15) as an active ingredient.
(22) Use of the quinoline derivative or the pharmaceutically acceptable salt thereof according to any one of above (1) to (8) for the manufacture of a PDE10A inhibitor.
(23) Use of the quinoline derivative or the pharmaceutically acceptable salt thereof according to any one of above (9) to (15) for the manufacture of a PDE10A inhibitor.
(24) Use of the quinoline derivative or the pharmaceutically acceptable salt thereof according to any one of above (1) to (8) for the manufacture of an agent for treating and/or preventing a disease caused by enhancing the activity of PDE10A.
(25) Use of the quinoline derivative or the pharmaceutically acceptable salt thereof according to any one of above (9) to (15) for the manufacture of an agent for treating and/or preventing a disease caused by enhancing the activity of PDE10A.
(26) Use of the quinoline derivative or the pharmaceutically acceptable salt thereof according to any one of above (9) to (15) for the manufacture of an agent for treating and/or preventing dyskinesia.
(27) Use of the quinoline derivative or the pharmaceutically acceptable salt thereof according to any one of above (9) to (15) for the manufacture of an antitumor agent.
(28) A method for treating a disease caused by enhancing the activity of PDE10A, which comprises administering an effective amount of the quinoline derivative or the pharmaceutically acceptable salt thereof according to any one of above (1) to (8).
(29) A method for treating a disease caused by enhancing the activity of PDE10A, which comprises administering an effective amount of the quinoline derivative or the pharmaceutically acceptable salt thereof according to any one of above (9) to (15).
(30) A method for treating dyskinesia, which comprises administering an effective amount of the quinoline derivative or the pharmaceutically acceptable salt thereof according to any one of above (9) to (15).
(31) A method for treating a malignant tumor, which comprises administering an effective amount of the quinoline derivative or the pharmaceutically acceptable salt thereof according to any one of above (9) to (15).
(32) Use of a compound having PDE10A inhibitory activity or a pharmaceutically acceptable salt thereof for the manufacture of an agent for treating and/or preventing dyskinesia.
(33) A method for treating dyskinesia, which comprises administering an effective amount of a compound having PDE10A inhibitory activity or a pharmaceutically acceptable salt thereof.

In the definitions for each group of general formulas (I) and (IA):
(i) Examples of the lower alkyl moiety of lower alkyl, lower alkoxy, mono-lower alkylamino and di-lower alkylamino include, for example, linear or branched alkyl having from 1 to 10 carbon atoms, such as methyl, ethyl, propyl, isopropyl, butyl, isobutyl, sec-butyl, tert-butyl, pentyl, isopentyl, neopentyl, hexyl, heptyl, octyl, isooctyl, nonyl, decyl and the like. The two lower alkyl moieties in di-lower alkyl amino may be the same or different.
(ii) Examples of the cycloalkyl include, for example, cycloalkyl having from 3 to 8 carbon atoms, such as cyclopropyl, cyclobutyl, cyclopentyl, cyclohexyl, cycloheptyl, cyclooctyl and the like.
(iii) Examples of the alkylene moiety of hydroxy lower alkyl include the same as the foregoing lower alkyl (i) from which one hydrogen atom is removed.
(iv) Examples of the lower alkanoyl include, for example, linear or branched alkanoyl having from 1 to 7 carbon atoms, such as formyl, acetyl, propionyl, butylyl, isobutylyl, valeryl, isovaleryl, pivaloyl, hexanoyl, heptanoyl and the like.
(v) Examples of the aryl include, for example, aryl having from 6 to 14 carbon atoms, such as phenyl, naphthyl, anthryl and the like.
(vi) Examples of the heterocyclic group include an alicyclic heterocyclic group and an aromatic heterocyclic group.
   Examples of the aromatic heterocyclic group include a 5-membered or 6-membered monocyclic aromatic heterocyclic group containing at least one atom selected from a nitrogen atom, an oxygen atom and a sulfur atom, a dicyclic or tricyclic condensed aromatic heterocyclic group comprising 3- to 8-membered rings and containing at least one atom selected from a nitrogen atom, an oxygen atom and a sulfur atom, and the like, for example, pyridyl, pyrazinyl, pyrimidinyl, pyridazinyl, benzoimidazolyl, 2-oxobenzoimidazolyl, benzotriazolyl, benzofuryl, benzothienyl, purinyl, benzooxazolyl, benzothiazolyl, benzodioxolyl, indazolyl, indolyl, isoindolyl, purinyl, quinolyl, isoquinolyl, phthalazinyl, naphthyllysinyl, quinoxalinyl, pyrrolyl, pyrazolyl, quinazolinyl, cinnolinyl, triazolyl, tetrazolyl, imidazolyl, oxazolyl, isooxazolyl, thiazolyl, isothiazolyl, thienyl, furyl and the like.
   Examples of the alicyclic heterocyclic group include a 5-membered or 6-membered monocyclic alicyclic heterocyclic group containing at least one atom selected from a nitrogen atom, an oxygen atom and a sulfur atom, a dicyclic or tricyclic condensed alicyclic heterocyclic group comprising 3- to 8-membered rings and containing at least one atom selected from a nitrogen atom, an oxygen atom and a sulfur atom, and the like, for example, pyrrolidinyl, 2,5-dioxopyrrolidinyl, thiazolidinyl, oxazolidinyl, piperidinyl, piperidino, piperazinyl, homopiperazinyl, homopiperidyl, homopiperidino, morpholinyl, morpholino, thiomorpholinyl, thiomorpholino, pyranyl, tetrahydropyridyl, tetrahydropyranyl, tetrahydrofuranyl, dihydrobenzofuranyl, tetrahydroquinolyl, tetrahydroisoquinolyl, octahydroquinolyl, indolinyl and the like.
(vii) Halogen means each atom of fluorine, chlorine, bromine and iodine.
(viii) Example of the condensed ring formed together with two carbon atoms on roots (thereof) include cycloalkane condensed with a benzene ring and the like.
   Examples of the cycloalkane moiety of the cycloalkane condensed with a benzene ring include, for example, cycloalkane having from 5 to 8 carbon atoms, such as cyclopentane, cyclohexane, cycloheptane, cyclooctane and the like. Examples of cycloalkane condensed with the benzene ring include indane, 1,2,3,4-tetrahydronaphthalene, 6,7,8,9-tetrahydro-5H-benzocycloheptene, 5,6,7,8,9,10-hexahydrobenzocyclooctene and the like.
(ix) The substituents in the substituted lower alkyl, the substituted lower alkoxy, the substituted cycloalkyl and the substituted lower alkanoyl may be the same or different, and include, for example, 1 to 3 substituent(s), such as halogen, hydroxy, carboxy, cyano, cycloalkyl, substituted or unsubstituted lower alkoxy, substituted or unsubstituted lower alkanoyl, substituted or unsubstituted aryl, a substituted or unsubstituted heterocyclic group, -NR¹⁰R¹¹ (wherein R¹⁰ and R¹¹ may be the same or different and each represents a hydrogen atom, substituted or unsubstituted lower alkyl or substituted or unsubstituted aryl) and the like. The position of the substituent(s) is not particularly limited.
   Herein, the halogen, the cycloalkyl, the lower alkyl moiety of the lower alkyl and the lower alkoxy, the lower alkanoyl, the aryl and the heterocyclic group have the same meanings as those of aforementioned halogen (vii), cycloalkyl (ii), lower alkyl (i), lower alkanoyl (iv), aryl (v) and heterocyclic group (vi) respectively.
   Also, herein the substituents (a) in the substituted lower alkoxy, the substituted lower alkyl and the substituted lower alkanoyl may be the same or different, and include, for example, 1 to 3 substituent(s), such as hydroxy, halogen and the like. And the substituents (b) in the substituted aryl and the substituted heterocyclic group may be the same or different, and include, for example, 1 to 3 substituent(s), such as hydroxy, halogen, lower alkyl, lower alkoxy, lower alkanoyl, aryl and the like. Herein, the halogen, the lower alkyl, the lower alkyl moiety of the lower alkyl and the lower alkoxy, the lower alkanoyl and the aryl have the same meanings as those of aforementioned halogen (vii), lower alkyl (i), lower alkanoyl (iv) and aryl (v) respectively.
(x) The substituents of the substituted aryl may be the same or different, and include, for example, 1 to 3 substituent(s), such as carboxy, hydroxy, halogen, substituted or unsubstituted lower alkyl, substituted or unsubstituted lower alkoxy, substituted or unsubstituted lower alkanoyl, substituted or unsubstituted aryl, a substituted or unsubstituted heterocyclic group and the like. The position of the substituent(s) is not particularly limited.
   Herein, the halogen, the lower alkyl moiety of the lower alkyl and the lower alkoxy, the lower alkanoyl, the aryl and the heterocyclic group have the same meanings as those of aforementioned halogen (vii), lower alkyl (i), lower alkanoyl (iv), aryl (v) and heterocyclic group (vi) respectively. Also, herein the substituents (c) in the substituted aryl include a substituted or unsubstituted lower alkyl [the lower alkyl has the same meaning as that of aforementioned lower alkyl (i), and the substituents in the substituted lower alkyl have the same meaning as that of the substituents (ix) listed in the definition for substituents in the aforementioned substituted lower alkyl] and the like as well as the group listed in the definition for substituents (ix) in the aforementioned substituted lower alkyl. Furthermore, herein the substituents in the substituted lower alkyl, the substituted lower alkoxy and the substituted lower alkanoyl have the same meaning as that of substituents (a) in the aforementioned substituted lower alkoxy and the like, and the substituents in the substituted heterocyclic group have the same meaning as that of substituents (c) in the aforementioned substituted aryl.
(xi) The substituents in the substituted heterocyclic group, the substituted piperazinyl, the substituted piperazin-1-yl, the substituted condensed ring formed together with carbon atoms on roots (thereof), cycloalkane condensed with a substituted benzene ring formed together with two carbon atoms on roots thereof, the substituted biphenylyl and the substituted biphenyl-4-yl may be the same or different, and include 1 to 3 substituent(s) such as carboxy, hydroxy, halogen, substituted or unsubstituted lower alkyl, substituted or unsubstituted lower alkoxy, substituted or unsubstituted lower alkanoyl, substituted or unsubstituted aryl, a substituted or unsubstituted heterocyclic group and the like. The position of the substituent(s) is not particularly limited.

Herein, the halogen, the lower alkyl moiety of the lower alkyl and the lower alkoxy, the lower alkanoyl, the aryl and the heterocyclic group have the same meanings as those of the aforementioned halogen (vii), lower alkyl (i), lower alkanoyl (iv), aryl (v) and heterocyclic group (vi) respectively.

Also, herein the substituents in the substituted lower alkyl, the substituted lower alkoxy and the substituted lower alkanoyl have the same meanings as that of the groups (ix) listed in the definition for substituents of the aforementioned substituted lower alkyl and the like, and the substituents in the substituted aryl and the substituted heterocyclic group have the same meaning as that of substituents (b) in the aforementioned substituted heterocyclic group.

Compounds represented by formula (I) or formula (IA) are referred to as compounds (I) or compounds (IA) respectively. The compounds having the other numbers are referred in the same manner.

A pharmaceutically acceptable salt of compound (I) or (IA) includes acid addition salt, metal salt, ammonium salt, organic amine addition salt, amino acid addition salt and the like which is pharmaceutically acceptable.

Examples of the pharmaceutically acceptable acid addition salt of compound (I) or compound (IA) include an inorganic acid salt such as a hydrochloride, a hydrobromide, a sulfate, a nitrate and a phosphate and the like, an organic acid salt such as an acetate, a benzenesulfonate, a benzoate, a citrate, a fumarate, a gluconate, a lactate, a maleate, a malate, an oxalate, a methanesulfonate and a tartrate, and the like. Example of the pharmaceutically acceptable metal salt includes an alkali metal salt such as a sodium salt and a potassium salt, an alkaline earth metal salt such as a magnesium salt and a calcium salt, an aluminum salt, a zinc salt and the like. Example of the pharmaceutically acceptable ammonium salt includes a salt of ammonium, tetramethylammonium or the like. Example of the pharmaceutically acceptable organic amine addition salt includes an addition salt of morpholine, piperidine or the like. Example of the pharmaceutically acceptable amino acid addition salt includes an addition salt of glycine, phenylalanine, lysine, aspartic acid, glutamic acid or the like.

Next, processes for preparing compound (I) and compound (IA) are described below.

In the preparing process as shown below, when the defined groups change under the reaction conditions in the following processes or the process is inappropriate in carrying out the processes, the preparation can be easily carried out by applying methods commonly used in organic synthetic chemistry, for example, protection or deprotection of functional groups [for example, Protective Groups in Organic Synthesis, third edition, T. W. Greene, John Wiley & Sons Inc. (1999)] and the like. Further, the order of the reaction step such as introduction of a substituent may be changed if neccessary.

Compounds (I) can be prepared by, for example, the following steps.

### Process 1

Among compound (I), compound (I-a) in which R¹ is carboxy and R³ is R^{3a} [wherein R^{3a} represents substituted or unsubstituted aryl (the aryl has the same meaning as that of the aforementioned aryl (v) and the substituents in the substituted aryl has the same meaning as that of the substituents (x) in the aforementioned substituted aryl) substituted or unsubstituted aryl (the aryl has the same meaning as that of aforementioned aryl (v) and the substituents in the substituted aryl has the same meaning as that of the substituents (c) in the aforementioned substituted aryl)] can be prepared by the following reaction steps. 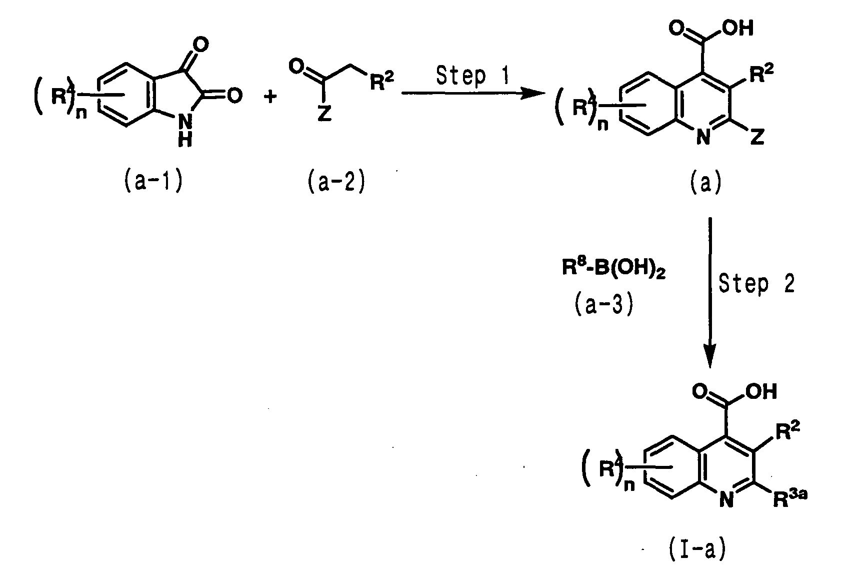 [wherein Z represents substituted or unsubstituted monohalogenated aryl (a halogen moiety of the monohalogenated aryl means each atom of chlorine, bromine and iodine, the aryl moiety of the monohalogenated aryl has the same meaning as that of aforementioned aryl (v), and the substituents in the substituted monohalogenated aryl has the same meaning as that of the substituents (x) in the aforementioned substituted aryl (excluding halogen)), or substituted or unsubstituted trifluoromethanesulfonyloxyaryl (the aryl moiety of the trifluoromethanesulfonyloxyaryl has the same meaning as that of aforementioned aryl (v), the substituents in the substituted trifluoromethanesulfonyloxyaryl has the same meaning as that of the substituents (x) in the aforementioned substituted aryl (excluding halogen)), R⁸ represents substituted or unsubstituted aryl (the aryl has the same meaning as that of aforementioned aryl (v), and the substituents in the substituted aryl has the same meaning as that of the substituents (c) in the aforementioned substituted aryl), and n, R², R^{3a} and R⁴ have the same meanings as those aforementioned respectively].

### Step 1:

Compound (a) can be obtained by condensation of compound (a-1) with 1 to 2 equivalents of compound (a-2) [see Pfitzinger reaction: J. Org. Chem., vol. 18, p. 1209 (1953) and the like].

For example, compound (a-1) is reacted with 1 to 2 equivalents of compound (a-2) in a solvent such as ethanol or methanol containing an aqueous solution of a base such as sodium hydroxide, potassium hydroxide, aqueous ammonia or the like at a temperature between 25°C and a boiling point of the solvent used for 5 minutes to 24 hours. This reaction mixture is acidified with a mineral acid such as hydrochloric acid or an organic acid such as acetic acid, whereby compound (a) can be obtained.

Compound (a-1) as a starting material can be obtained by the method described in Adv. Het. Chem., vol. 18, p. 1 (1975) and the like or similar methods thereto. Compound (a-2) in which Z is monohalogenated aryl can be obtained by the method described in J. Org. Chem., vol. 27, p. 70 (1962) and the like or similar methods thereto. Compound (a-2) in which Z is trifluoromethanesulfonyloxyaryl can be obtained as a commercial product or by the method described in Japanese Published Unexamined Patent Application No. 161075/82 and the like, or similar methods thereto.

### Step 2:

Compound (I-a) can be obtained by subjecting compound (a) prepared in step 1 and a corresponding boronic acid reagent (compound (a-3)) to the Suzuki-Miyaura reaction (Suzuki-Miyaura reaction: see Chem. Rev., vol. 95, p. 2457 (1995) and the like).

For example, compound (I-a) is obtained by reacting compound (a) with 1 to 2 equivalents of compound (a-3) in an inert solvent in the presence of a palladium catalyst and 1 to 5 equivalents of a base at a temperature between 25°C and a boiling point of the solvent used for 5 minutes to 24 hours.

Examples of the palladium catalyst include bis(tri-o-tolylphosphine)palladium (II) dichloride, bis(triphenylphosphine)palladium (II) dichloride, tetrakis(triphenylphosphine)palladium and the like.

Examples of the base include pyridine, triethylamine, potassium carbonate, sodium carbonate, sodium hydrogencarbonate, sodium hydroxide and the like.

Examples of the inert solvent include methanol, ethanol, chloroform, N,N-dimethylformamide (DMF), dioxane and the like.

Compound (a-3) as a starting material can be obtained as a commercial product or by the method described in Organometallics, vol. 2, p. 1316 (1983) and the like, or similar methods thereto.

### Process 2

Among compound (I), compound (I-b) or compound (I-c) in which R¹ is hydroxymethyl or methyl and R³ is R^{3a} (wherein R^{3a} has the same meanings as that above-mentioned) can be prepared by the following reaction steps respectively. 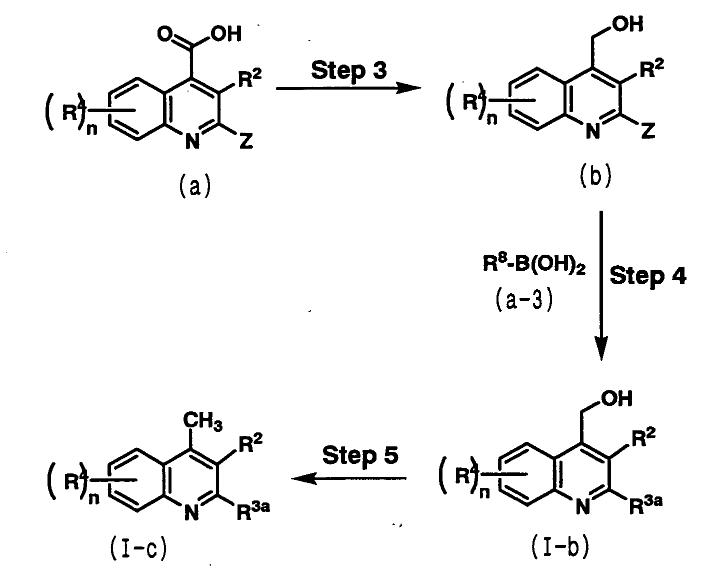 [wherein Z, n, R², R^{3a}, R⁴ and R⁸ have the same meanings as those above-mentioned respectively.]

### Step 3:

Compound (b) can be obtained by conversion of a carboxyl group of compound (a) prepared in step 1 to an ester such as a methyl ester, an ethyl ester, a hydroxybenzotriazole ester or the like by a method well known in the field of organic synthetic chemistry [see Synthesis, p. 929 (1985) and the like] and subsequent subjecting the resulting ester to a reduction reaction [see Shin Jikken Kagaku Koza, 4th edition, 15 (Oxidation and Reduction II), Maruzen, pp. 158 and 179 (1977) and the like].

For example, compound (a) is reacted with 1 to 2 equivalents of N,N-dimethylchloromethaneiminium chloride in an inert solvent such as dichloromethane, 1,2-dichloroethane, chloroform, DMF, dioxane or the like in the presence of 1 to 5 equivalents of a base at a temperature between -20°C and a boiling point of the solvent used for 5 minutes to 24 hours to obtain corresponding N,N-dimethylacyloxymethaneiminium chloride. Examples of the base used here include pyridine, triethylamine, N-methylmorpholine, potassium carbonate, sodium carbonate, sodium hydrogencarbonate, sodium hydroxide and the like. The resulting N,N-dimethylacyloxymethaneiminium chloride is reacted with 1 to 2 equivalents of hydroxybenzotriazole monohydrate in a solvent in the presence of 1 to 5 equivalents of a base at a temperature between -20°C and a boiling point of the solvent used for 5 minutes to 24 hours to obtain corresponding hydroxybenzotriazole ester. Examples of the solvent used here include dichloromethane, DMF and the like. Examples of the base include pyridine, triethylamine, N-methylmorpholine, potassium carbonate, sodium carbonate, sodium hydrogencarbonate, sodium hydroxide and the like.

Compound (a) is, for example, treated with a corresponding alcohol such as methanol, ethanol or the like in the presence of an acid catalyst such as sulfuric acid or the like without a solvent or in an inert solvent such as dichloromethane, 1,2-dichloroethane, chloroform, DMF, dioxane or the like to obtain a corresponding ester such as a methyl ester, an ethyl ester or the like.

Also, Compound (a) is, for example, reacted with thionyl chloride in the presence or absence of a base without a solvent or in an inert solvent such as benzene, toluene or the like, and then treated with a corresponding alcohol such as methanol, ethanol or the like to obtain a corresponding ester such as a methyl ester, an ethyl ester or the like.

Examples of the base used here include pyridine, triethylamine, N-methylmorpholine, potassium carbonate, sodium carbonate, sodium hydrogencarbonate, sodium hydroxide and the like.

Then, the resulting hydroxybenzotriazole ester or the resulting ester is treated in a protic polar solvent or an aprotic solvent in the presence of, for example, 2 to 5 equivalents of a reducing agent at a temperature between 0°C and a boiling point of the solvent used for 5 minutes to 24 hours to obtain compound (b).

Examples of the protic polar solvent include methanol, ethanol, isopropyl alcohol and the like, and examples of the aprotic solvent include tetrahydrofuran (THF), diethyl ether and the like.

Examples of the reducing agent include sodium borohydride, aluminum lithium hydride (LAH) and the like. When LAH is used, an aprotic solvent is used preferably.

N,N-dimethylchloromethaneiminium chloride as a starting material can be prepared from oxalyl chloride and DMF by, for example, the method described in Synthesis, p. 929 (1985) and the like or similar methods thereto.

### Step 4:

In a manner similar to that of step 2, compound (I-b) can be obtained from compound (b) prepared in step 3 and compound (a-3).

### Step 5:

Compound (I-c) can be obtained by conversion of a hydroxymethyl group of compound (I-b) prepared in step 4 to a chloromethyl group [see Jikken Kagaku Koza, 4th edition, 19 (Organic Synthesis I Hydrocarbon Halogenated compound), Maruzen, p. 444 (1992)] and subsequent subjecting the resulting product to a reduction reaction [see Shin Jikken Kagaku Koza, 4th edition, 15 (Oxidation and reduction II), Maruzen, p. 181 (1977)].

For example, compound (I-b) is converted to a corresponding chloromethyl derivative by a reaction with 5 to 10 equivalents of thionyl chloride at a temperature between 0°C and a boiling point of thionyl chloride for 5 minutes to 24 hours, and the chloromethyl derivative is then treated with 2 to 5 equivalents of sodium borohydride or the like in an inert solvent at a temperature between 0°C and a boiling point of the solvent used for 5 minutes to 24 hours to obtain compound (I-c).

Examples of the inert solvent include dioxane and the like.

### Process 3

Among compound (I), compound (I-k) in which R³ is hydroxy or halogen can be obtained by the method described in WO 97/00074 or a method corresponding thereto.

### Process 4

Among compound (I), compound (I-d), compound (I-e) or compound (I-f) in which R¹ is methoxycarbonyl, carboxy or hydroxymethyl and R³ is substituted or unsubstituted piperazin-1-yl can be prepared by the following reaction steps. 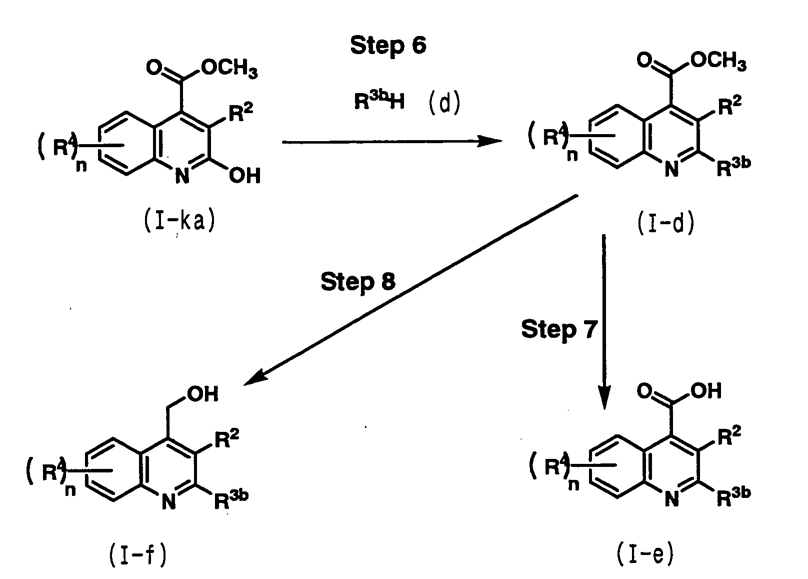 [wherein n, R² and R⁴ have the same meanings as those above-mentioned respectively, and R^{3b} represents substituted or unsubstituted piperazin-1-yl (the substituent in the substituted piperazin-1-yl has the same meaning as that of the substituents (xi) in the substituted piperazin-1-yl).]

### Step 6:

Among compound (I-k) prepared in process 3, a hydroxyl group of compound (I-ka) in which R¹ is methoxycarbonyl and R³ is hydroxy is converted to a trifluoromethanesulfonyloxy group by the method described in WO 97/00074 or by a method corresponding thereto. The resulting trifluromethanesulfonate is reacted with compound (d), whereby compound (I-d) can be obtained.

For example, compound (I-ka) can be converted to a trifluoromethanesulfonate derivative by a reaction with 1 to 3 equivalents of, for example, trifluoromethanesulfonic anhydride in an inert solvent in the presence of 1 to 3 equivalents of a base at a temperature between 0°C and a boiling point of the solvent used for 5 minutes to 24 hours. Examples of the base used here include pyridine, triethylamine, potassium carbonate, sodium carbonate, sodium hydrogencarbonate, sodium hydroxide and the like. Examples of the inert solvent include dichloromethane, 1,2-dichloroethane, chloroform, DMF, dioxane, acetonitrile and the like. Subsequently, the trifluoromethanesulfonate derivative is reacted with 1 to 3 equivalents of compound (d) in an inert solvent in the presence of 1 to 3 equivalents of a base at a temperature between 0°C and a boiling point of the solvent used for 5 minutes to 24 hours to obtain compound (I-d). Examples of the base used here include pyridine, triethylamine, potassium carbonate, sodium carbonate, sodium hydrogencarbonate, sodium hydroxide and the like. Examples of the inert solvent include dichloromethane, 1,2-dichloroethane, chloroform, DMF, dioxane, acetonitrile and the like.

Compound (d) as a starting material can be obtained as a commercial product (Aldrich or the like).

### Step 7:

Compound (I-e) can be prepared by subjecting compound (I-d) to a hydrolysis reaction [see Jikken Kagaku Koza, 4th edition, 22 (Organic Synthesis IV Acid·Amino acid·Peptide), Maruzen, p. 7 (1992)].

For example, compound (I-d) is treated in a solvent containing 1 to 1,000 equivalents of water in the presence of 1 to 10 equivalents of a base at a temperature between 0°C and a boiling point of the solvent used for 5 minutes to 24 hours to obtain compound (I-e).

Examples of the inert solvent include diethyl ether, tetrahydrofuran (THF), toluene and the like.

Examples of the base include pyridine, triethylamine, potassium carbonate, sodium carbonate, sodium hydrogencarbonate, sodium hydroxide and the like.

### Step 8:

Compound (I-f) can be obtained by subjecting compound (I-d) to a reduction reaction [see Shin Jikken Kagaku Koza, 4th edition, 15 (Oxidation and Reduction II), Maruzen, pp. 158-179 (1977)].

For example, compound (I-d) is treated in an inert solvent in the presence of 1 to 3 equivalents of a reducing agent such as LAH or the like at a temperature between 0°C and a boiling point of the solvent used for 5 minutes to 24 hours to obtain compound (I-f).

Examples of the inert solvent include diethyl ether, THF, toluene and the like.

### Process 5

Among compound (I), compound (I-h), compound (I-i) or compound (I-j) in which R¹ is carbamoyl, cyano or amino can be prepared by the following steps respectively. 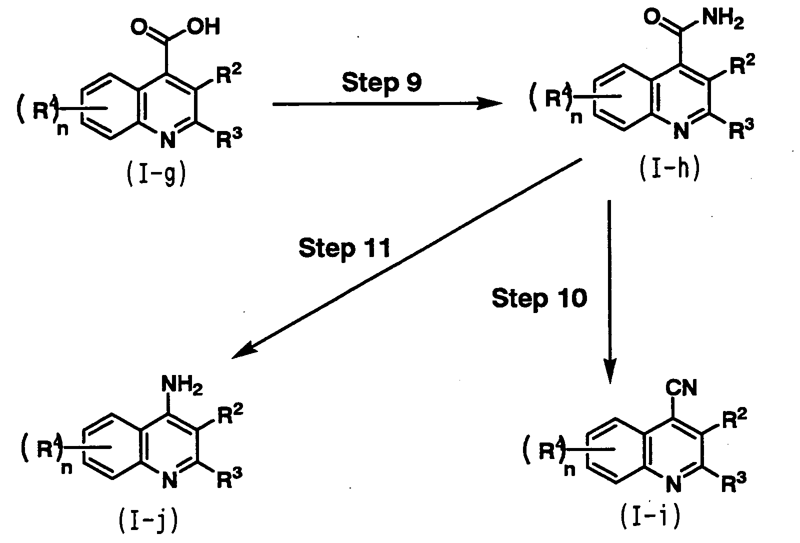 (wherein n, R², R³ and R⁴ have the same meanings as those above-mentioned respectively.)

### Step 9:

Compound (I-h) can be obtained by conversion of a carboxyl group of compound (I-g) prepared by the method described in Japanese Published Unexamined Patent Application No. 231289/98, Japanese Published Unexamined Patent Application No. 3144/96, WO 95/10505 and the like or similar methods thereto to a chlorocarbonyl group and subsequent treatment of the acid chloride with aqueous ammonia (see Japanese Published Unexamined Patent Application No. 231289/98 and the like).

For example, compound (I-g) can be converted to an acid chloride by a reaction with 1 to 10 equivalents of a chlorinating agent in an inert solvent such as benzene, toluene or the like in the presence or absence of a base at a temperature between 0°C and a boiling point of the solvent or the chlorinating agent used for 5 minutes to 24 hours. Examples of the base used include pyridine, triethylamine, potassium carbonate, sodium carbonate, sodium hydrogencarbonate, sodium hydroxide and the like. Examples of the chlorinating agent include thionyl chloride, phosphoryl chloride, oxalyl chloride and the like.

Subsequently, the acid chloride is treated with 1 to 5 equivalents of aqueous ammonia in an inert solvent at a temperature between 0°C and a boiling point of the solvent used for 5 minutes to 24 hours to obtain compound (I-h). Examples of the inert solvent include dichloromethane, 1,2-dichloroethane, chloroform, diethyl ether, THF, toluene, DMF, dimethyl sulfoxide and the like.

### Step 10:

Compound (I-i) can be obtained by treatment of compound (I-h) with, for example, a chlorinating agent and the like [see J. Am. Chem. Soc. vol., 70, p. 3315 (1948)].

For example, compound (I-h) is treated with 1 to 10 equivalents of a chlorinating agent at a temperature between 0°C and a boiling point of the chlorinating agent used for 5 minutes to 24 hours to obtain compound (I-i).

Examples of the chlorinating agent include thionyl chloride, phosphoryl chloride, oxalyl chloride and the like.

### Step 11:

Compound (I-j) can be obtained by treatment of the carbamoyl group of compound (I-h) with, for example, a hypochlorite, a hypobromite or the like [see Hoffman Rearrangement: Jikken Kagaku Koza, 4th edition, 20 (Organic Synthesis II Alcohol·Amine), Maruzen, p. 304 (1991)].

For example, compound (I-h) is treated with 1 to 5 equivalents of a hypochlorite, a hypobromite or the like in a protic polar solvent at a temperature between 0°C and a boiling point of the solvent used for 5 minutes to 24 hours to obtain compound (I-j).

Examples of the protic polar solvent include water, ethanol, methanol and the like.

### Process 6

Among compound (I), a compound in which R¹ is methoxycarbonyl, hydroxymethyl or methyl can also be prepared from compound (I-g) as a starting material used in process 5 by the methods described in steps 3 and 5 of process 2 respectively or similar methods thereto.

### Process 7

Compound (I) can be prepared by, for example, the following processes besides the processes described in above processes 1 to 6.

For example, among compound (I), a compound in which R¹ is substituted or unsubstituted lower alkyl, -C(=Y)R⁹ (wherein Y and R⁹ have the same meanings as those above-mentioned respectively), hydroxy, halogen, mono-lower alkylamino or di-lower alkylamino can be prepared by the method described in, for example, Japanese Published Unexamined Patent Application No. 3144/96, WO 95/10505 and the like, a method corresponding thereto or a process well known in the field of organic chemistry.

Furthermore, among compound (I), a compound in which R² and R³ form a substituted or unsubstituted condensed ring together with two carbon atoms on roots thereof can be produced by the method described in, for example, Bioorg. Med. Chem. Lett., vol. 8, p. 307 (1998), Japanese Published Unexamined Patent Application No. 306079/94 and the like, a method corresponding thereto or a method well known in the field of organic chemistry.

In compound (I), the intermediates and the starting material, conversion of the functional groups and conversion of the functional groups contained in the substituents can be carried out according to other known methods [for example, the method described in Comprehensive Organic Transformations, second edition, R. C. Larock, John Wiley & Sons Inc. (1999) and the like] besides the aforementioned steps.

Further, compound (I) having desired functional groups on the desired positions can be obtained by carrying out a appropriate combination of the aforementioned processes and the like.

Also, compound (IA) can be prepared in the same manner as in the aforementioned process for compounds (I).

The intermediates and the desired compounds in the aforementioned processes can be isolated and purified by subjecting to separation and purification methods ordinarily used in organic synthetic chemistry, such as filtration, extraction, washing, drying, concentration, recrystallization, various chromatographies and the like. The intermediates may be subjected to the next reaction without particular purification.

Among compound (I) or (IA), stereoisomers such as a geometric isomer and an optical isomer may be existed. All possible isomers including these isomers and the mixtures of the isomers at any ratios can be used in the PDE10A isomer of the present invention.

To obtained a salt of compound (I) or (IA), when compound (I) or (IA) are obtained as a salt form, it may be purified as it is. When compound (I) or (IA) are obtained as a free form, it may be dissolved or suspended in an appropriate solvent and isolated and purified by addition of an acid or a base.

Compound (I) or (IA) and a pharmaceutically acceptable salt thereof may be existed in the form of adducts with water or various solvents. These adducts can also be used for the PDE10 inhibitor of the present invention.

Specific examples of compound (IA) obtained in the present invention are shown in Tables 1 to 3. However, the compounds of the present invention are not limited thereto.

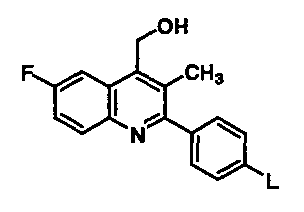

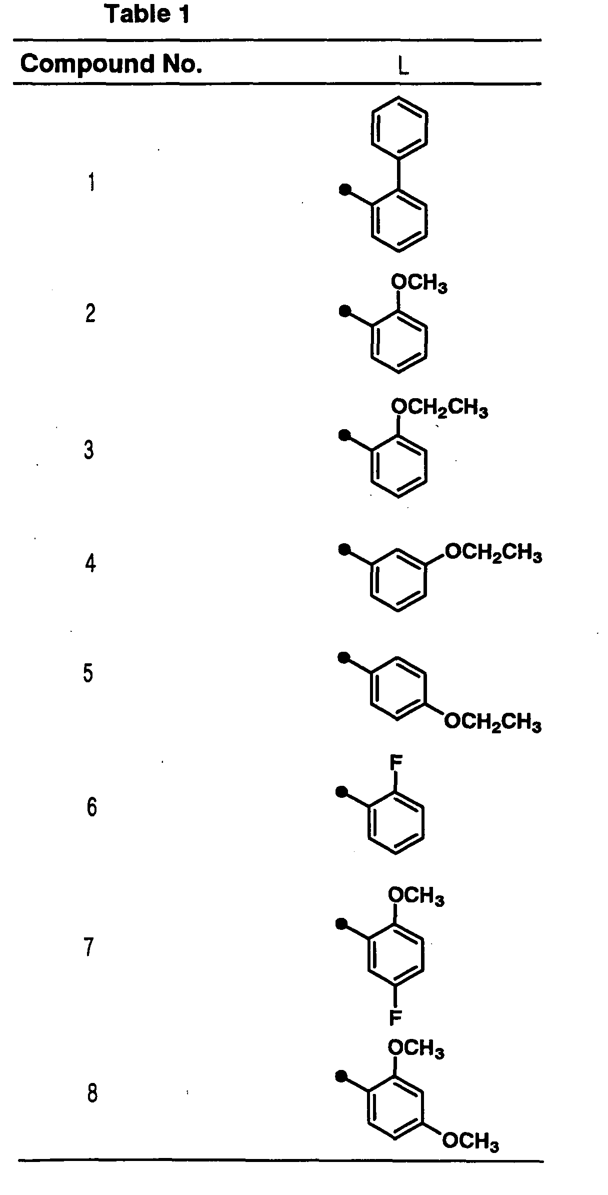

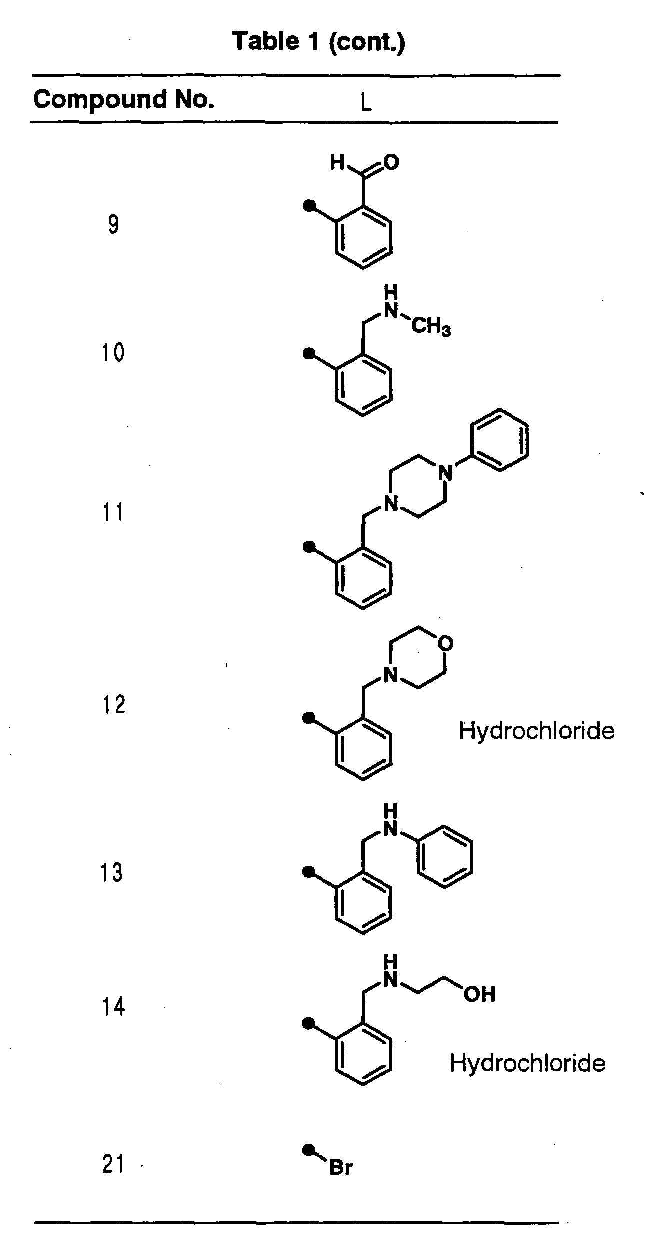

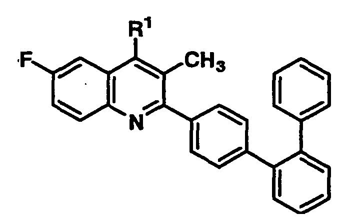

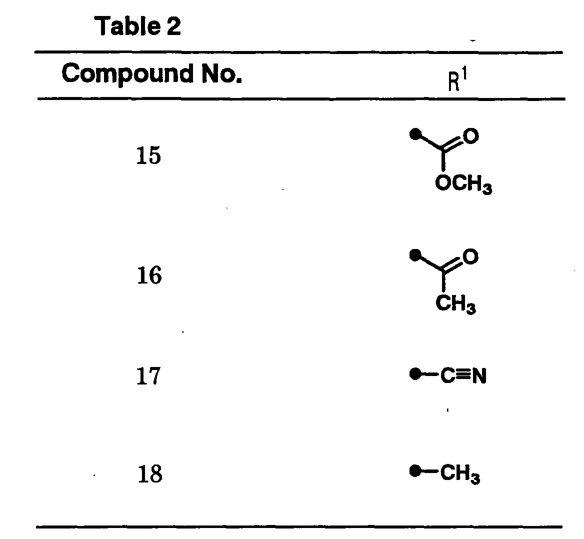

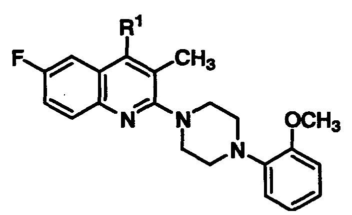

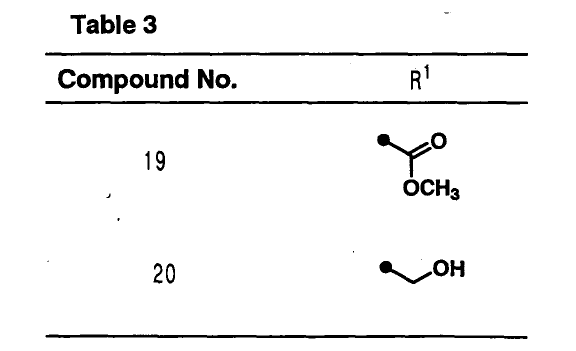

The compounds listed in Tables 1 to 3 are used for the PDE inhibitor of the present invention, and specific examples of compounds used in the present invention besides these compounds are shown in Table 4. However, the compounds used in the present invention are not limited thereto.

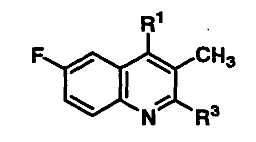

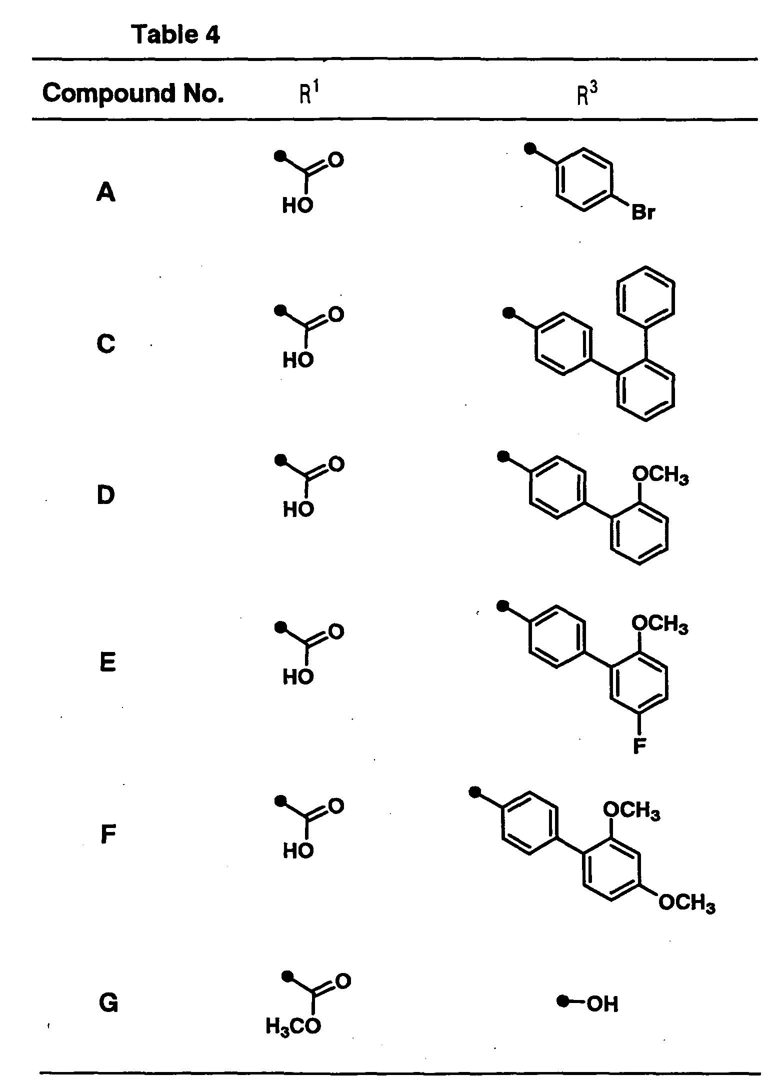

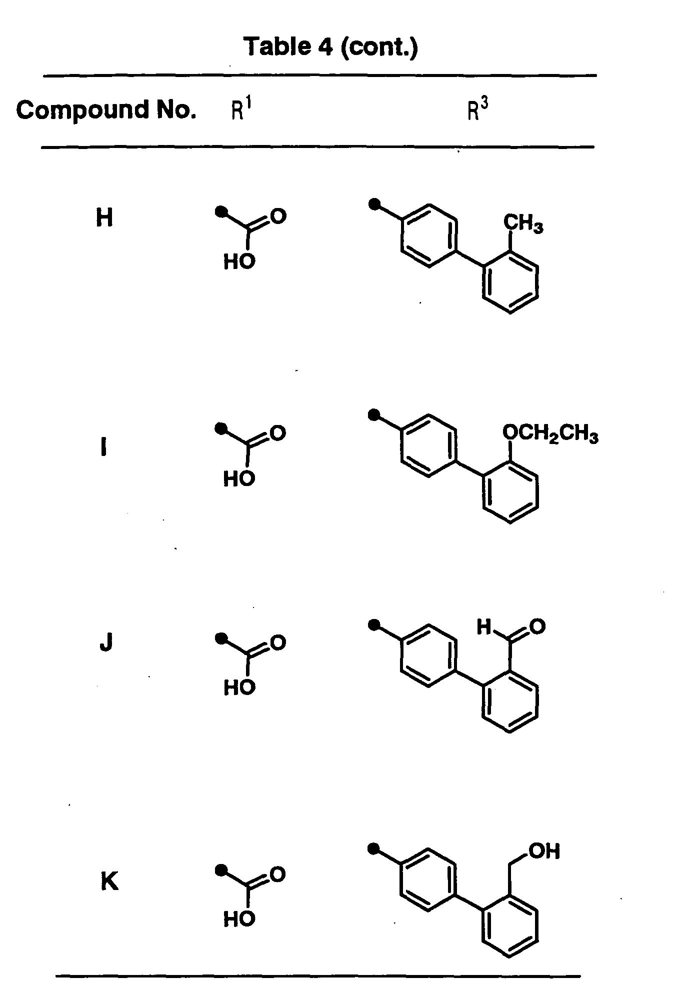

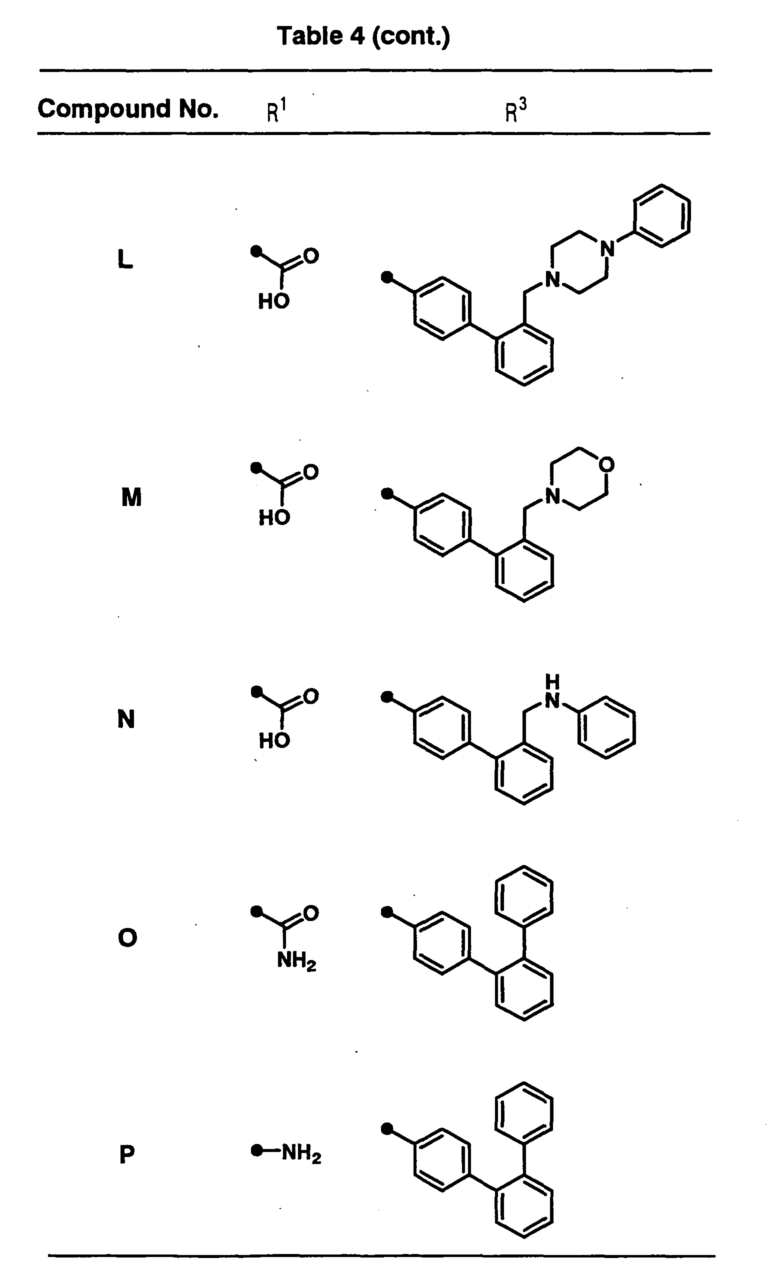

Next, pharmacological activities of typical compounds (I) are specifically described by test examples.

### Test Example 1:

### Activity of inhibiting PDE10A activity ([³H]cAMP decomposition inhibiting test)

PCR was performed using DNAs having base sequences described in SEQ ID NO. 1 and SEQ ID NO. 2 as primers and human kidney cDNA as a template to prepare a plasmid containing amplified fragments. The plasmid was cleaved with restriction endonucleases Pst I and Xba I to obtain a Pst I-Xba I fragment of 0.7 kb.

Meanwhile, Est clone (Cosmo·Bio, Tokyo) of GenBank, ACCESSION WO 4835 was cleaved with restriction endonucleases Kpn I and Pst I to obtain a Kpn I-Pst I fragment of 1.5 kb.

The resulting Pst I-Xba I fragment of 0.7 kb and Kpn I-Pst I fragment of 1.5 kb were subcloned in a Kpn I-Xba I site of pBluescript II KS(-) (STRATAGENE, La Jolla, CA, USA) to construct a plasmid having a cDNA fragment completely including a catalyst region of PDE10A. Subsequently, the plasmid was cleaved with restriction endonucleases Kpn I and Not I to obtain a Kpn I-Not I fragment of 2.2 kb.

The resulting Kpn I-Not I fragment of 2.2 kb and a linker having a BamH I-Kpn I cleavage surface prepared by synthetic DNA having base sequences described in SEQ ID NOS. 3 and 4 were subcloned in a BamH I-Not I site of pVL1393 (PharMingen, San Diego, CA, USA) to obtain a plasmid for preparation of Baculovirus. The plasmid contains a DNA encoding a peptide in which methionine, FLAG tag and a peptide having a 64th to 779th amino acid sequence of PDE10A [GenBank, ACCESSION NP 006652: Gene, vol. 234, p. 109 (1999), J. Biol. Chem., vol. 274, p. 18438 (1999)] are bound in the order.

The above gene recombinational manipulation was performed according to the method described in Molecular Cloning, 2nd edition, Cold Spring Harbor Laboratory Press, New York (1989).

The expression of PDE10A was conducted according to the Baculovirus expression vector system manual (PharMingen) using insect cells. As insect cells, Sf9 cells (Asahi Technoglass, Tokyo) were used. Sf9 cells were infected with a supernatant (co-transfection sup) containing PDE10A expression virus prepared according to the manual, and cultured at 27°C for 4 days. The cells were recovered, washed with a phosphate-buffered saline (PBS), and then suspended in an extraction solution [20 mmol/L Tris-acetate (pH 7.5), 2 mmol/L MgCl₂, 1 mmol/L dithiothreitol, 1 mmol/L ethylenediaminetetraacetic acid (EDTA), 0.25 mol/L sucrose, 250 unit/mL aprotinin, 40 µg/mL phenylmethylsulfonyl fluoride, and 1 µg/mL pepstatin A]. The cells were disrupted in ice with the maximum output under conditions of 5 seconds and five times using an ultrasonic disruption machine (TOMY model UR-200R, TOMY, Tokyo). A soluble fraction was obtained by centrifugation under 10,000 rpm of the fraction at 4°C for 30 minutes, and the resulting soluble fraction was used for measuring the PDE activity.

The PDE activity was measured according to the method described in Methods Enzymol., vol. 159, p. 457 (1988).

Using [³H]cAMP as a substrate, an enzyme reaction was conducted in a reaction solution containing 300 µL of 50 mmol/L N,N-bis(2-hydroxyethyl)-2-aminoethanesulfonic acid (pH 7.2), 1 mmol/L MgCl₂ and 0.1 mmol/L ethylene glycol-bis (β-aminoethyl ether)-N,N,N',N'-tetraacetic acid: EGTA). The concentration of the substrate was 0.2 µmol/L. To 95 µL of an enzyme dilute solution were added 5 µL of a test compound (DMSO solution) and 200 µL of a substrate solution. The mixture was incubated at 30°C for 20 minutes, and then HCl was added to stop the reaction. The reaction product (5'-AMP) was converted to adenosine using 5'-nucleotidase, and separated the unreacted product from the reactant by DEAE-Sephadex A-25 column (Amersham Pharmacia Biotech, Uppsala, Sweden). The elute was moved to scintillation vials, 6 mL of Ultima Gold (Packard Instrument, Meriden, CT, USA) was added, and the radioactivity was measured for 2 minutes using a liquid scintillation counter (Beckman LS6500, Beckman, Tokyo). A non-catalytic hydrolysis amount was an amount of [³H]cAMP decomposition when an enzyme dilute solution was not added. A decomposition amount by the soluble fraction was obtained by subtracting the non-catalytic hydrolysis amount from the total decomposition amount. The inhibitory activity of [³H]cAMP decomposition of test compounds is shown in Table 5 as a [³H]cAMP decomposition inhibition rate (%) and a concentration (IC₅₀) at which to inhibit [³H]cAMP decomposition by 50%.

**Table 5**

| Compound No. | [³H] cAMP decomposition Inhibition rate (%) (Test compound concentration 1 µmol/L) | IC₅₀(nmol/L) |
|---|---|---|
| C | 99 | 0.9 |
| D | 99 | 11 |
| H | 97 | 47 |
| I | 99 | 5.5 |
| J | 99 | 16 |
| K | 90 | NT |
| L | 99 | 21 |
| M | 98 | 27 |
| N | 99 | 13 |
| O | 94 | 33 |
| 1 | 99 | 4.2 |
| 2 | 97 | 66 |
| 3 | 97 | 76 |
| 4 | 83 | NT |
| 6 | 91 | 98 |
| 7 | 97 | 34 |
| 8 | 68 | NT |
| 11 | 90 | 173 |
| 12 | 96 | 68 |
| 13 | 90 | 103 |
| 16 | 80 | NT |
| 17 | 70 | 373 |
| 18 | 97 | 4.8 |
| NT = not performed | | |

### Test Example 2:

### Test for activity to hyperkinesia in 6-hydroxydopamine (6-OHDA)-treated rats

When the unilateral nigrostriatal pathway is lesioned with 6-OHDA, sensitivity of dopamine receptors in the striatum on the lesioned side is increased. When a dopamine activity enhancing substance such as L-DOPA is administered thereto, a rotating behavior takes place on the side opposite to the lesioned side. This animal model has been long positioned as an excellent test model of Parkinson's disease, and has been used for research of therapeutic agent for Parkinson' s disease [Neurol. Neurobiol. vol. 33, p. 1 (1987)]. Repetitive administration of L-DOPA to 6-OHDA-treated rats has been considered to be a similar model of dyskinesia which is a side effect induced by long-term administration of L-DOPA as a therapeutic agent for Parkinson's disease [Exp. Neurol. vol. 151, p. 334 (1998)].

In this test, 6-week-old male rats (strain's name: Cr j:CD(SD)IGS) were purchased from Japan Charles River, and used after conditioned breeding for 6 days. After the rat was anesthetized with pentobarbital sodium (40 mg/kg, intravenous administration), a solution which is dissolving 8 µg of 6-hydroxydopamine hydrobromide (6-OHDA hydrobromide) in 2 µL of physiological saline containing 0.05% ascorbic acid was injected in the medial forebrain bundle over 3 minutes to destroy unilateral nigrostriatal pathway. For protecting noradrenaline neurons, 25 mg/kg of deciprasmine hydrochloride was intraperitoneally administered 30 minutes before operation.

After 7 days from the injection of 6-OHDA, 0.1 mg/kg of apomorphine which is a dopamine agonist was subcutaneously administered to the rats. Individuals in which the rotating behavior was observed were used in the following test as Parkinson's disease model rats in which the lesion was successful.

After withdrawal of the administration of apomorphine for 14 days, L-DOPA and benserazide were orally administered to the rats at doses of 100 mg/kg and 25 mg/kg respectively to induce hyperkinesia. At this time, the number of rotations was measured for 60 minutes immediately after the administration. On the basis of the results of measuring the number of rotations, the rats were divided into A group and B group, each group consisting of 8 rats, such that there was no statistically significant difference in average number of rotations between the groups. In the measurement of the number of rotations, an automatic rotation measuring device was used, and rotation at 180° was estimated as 1 count. Consequently, the average number of rotations for 60 minutes was 1,389 ± 196 in A group and 1,396 ± 212 in B group.

After 7 days from the dividing into groups, the number of rotations for 60 minutes was measured by similar manner as that of above-mentioned. A vehicle (0.5% methylcellulose aqueous solution) was intraperitoneally administered to A group, and 50 mg/kg of the test compound was intraperitoneally administered to B group. Immediately after the administration, 30 mg/kg of L-DOPA and 7.5 mg/kg of benserazide were orally administered to A and B groups, and the number of rotations for 60 minutes was measured every 5 minutes.

In the vehicle administration group (A group), the total number of rotations for 60 minutes was 1,298 ± 193. Meanwhile, in the group (B group) to which compound 12 was administered, it was 48 ± 20. The vehicle administration group and the test compound administration group were statistically compared in the total number of rotations by the student's test. Consequently, the difference was a significant difference with a significance level of less than 0.1%.

From the results above-mentioned, it was revealed that the administration of compounds (I) inhibits hyperkinesia in 6-OHDA-treated rats. Therefore, it is considered that the PDE10 A inhibitors including compounds (I) can reduce dyskinesia.

### Test Example 3: Cell growth inhibitory activity

Cell growth inhibitory activity of compound 12 was examined on MDA-MB-231 (human breast cancer cell: American Type Culture Collection) using an in-vitro cell growth assay [3-(4,5-dimethylthiazol-2-yl)-2,5-diphenyl tetrazolium bromide (MTT)]-type calorimetric assay [Leukemia, vol. 7, p. 1637, (1993)].

MDA-MB-231 (2.5 x 10⁴ cells/mL, 270 µL) was inoculated on a 48-well plate, and cultured overnight. In the culture, Leibovitz's L-15 (Gibco) containing 10% fetal calf serum (Hi-Clone), 0.05 units/mL of penicillin (Gibco) and 0.05 µg/mL of streptomycin (Gibco) was used as a culture medium. 30 µL of compound 12 dissolved in the same medium containing 0.1% dimethyl sulfoxide (DMSO; Wako Pure Chemical Industries) was added to each well. After 3 days of culture, 30 µL of MTT (Dojin Kagaku Kenkyusho, adjusted to a concentration of 5 mg/mL with PBS) was added to each well. After 4 hours of culture, 900 µL of DMSO was added to dissolve the cells. The cell solution was moved to a 96-well plate in amounts of 200 µL each, and fluorescence intensity OD₅₉₀₋₆₃₀ was measured with an automated microplate reader EL340 (Biotech Instruments). The inhibition rate of the cell growth was calculated from the following equation, wherein OD (sample) is fluorescence intensity (OD₅₉₀₋₆₃₀) of the group being added compound 12 (medium containing compound 12 and 0.1% DMSO), OD (control) is fluorescence intensity (OD₅₉₀₋₆₃₀) of the group being absent of compound 12 (medium containing 0.1% DMSO alone was added) and OD (blank) is fluorescence intensity (OD₅₉₀₋₆₃₀) of the same medium containing 0.1% DMSO.

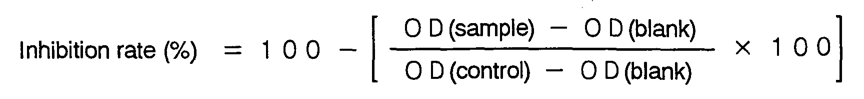

Compound 12 showed 39% of inhibitory activity to growth of MDA-MB-231 at 10 µmol/L.

From the results, it was suggested that compound (I) or (IA), or the pharmaceutically acceptable salts thereof are useful as an agent for treating human breast cancer.

In view of the above-mentioned, compound (I) or (IA), or the pharmaceutically acceptable salts thereof have the PDE10A inhibitory activity, and are considered to be useful for treatment of various diseases caused by enhancing the activity of PDE10A [for example, Parkinson' s disease, tremor, dyskinesia (L-DOPA-induced dyskinesia, tardive dyskinesia, myoclonus, tic or Tourette syndrome), Huntington disease, dystonia, an anxiety disorder (panic attack and panic disorder, phobia, obsessive compulsive disorder, post-traumatic stress disorder, acute stress disorder, generalized anxiety disorder, anxiety due to physical handicap or substance), a mood disorder (depression, dysthymic disorder, bipolar disorder or mood cyclothymia disorder), congitive impairment (aphasia, apraxia, agnosia, delirium, dementia or amnesia), an emotional disturbance disorder induced by drug abuse or stopping of drug use, schizophrenia and its-related disorder (short-term psychotic disorder, schizoid disorder, schizoid-emotional disorder or delusional disorder), a cerebrovascular disease (transient cerebral ischemic attack (TIA), ischemic attack, intracranial hemorrhage or subarachnoidal hemorrhage), head injury, hypogonadism (impotence), diabetes, a ischemic heart disease (angina pectoris, acute coronary syndrome or old myocardial infarction), a renal disease (acute renal insufficiency, chronic renal insufficiency or glomerulonephritis), peripheral a vascular disease (peripheral arterial occlusion, occlusive thromboangiitis, Raynaud disease and Raynaud phenomenon, acrocyanosis or erythromelalgia), hypertension, urinary incontinence (transient urinary incontinence or hemostatic urinary incontinence), an autoimmune disease (chronic rheumatoid arthritis, systemic lupus erythematosus, osteoarthritis, multiple sclerosis or psoriasis), a pulmonary disease (chronic obstructive pulmonary disease, chronic bronchitis, pulmonary emphysema or asthma), an allergic disease (itching, contact dermatitis, atopic dermatitis, allergic rhinitis or allergic conjunctivitis), a disease of the digestive system, osteoporosis, contraception, pain (acute postoperative pain, cancer pain, neuropathic pain or phychogenic pain syndrome), a malignant tumor or the like.]

Compound (I) or (IA), or the pharmaceutically acceptable salt thereof can be administered by itself as it is, however, preferably it is usually provided as various pharmaceutical preparations. Further, these pharmaceutical preparations are used in animals or humans.

The pharmaceutical preparations according to the present invention can contain compound (I) or (IA), or the pharmaceutically acceptable salt thereof as an active ingredient either solely or as a mixture with any other active ingredient for treatment. The pharmaceutical preparations are prepared by mixing the active ingredient with one or more of pharmaceutically acceptable carriers by any method well known in the technical field of pharmaceutical science.

As for an administration route, it is preferred to use the most effective administration route in treatment. Oral administration or parenteral administration such as intravenous administration and the like can be mentioned.

Examples of a dosage form include tablets, capsules, injections and the like.

Examples of the carrier for preparations used include lactose, mannitol, glucose, hydroxypropyl cellulose, starch, magnesium stearate, sorbitan fatty acid ester, glyceric acid ester, polyvinyl alcohol, distilled water for injection, physiological saline, propylene glycol, polyethylene glycol, ethanol and the like. Also, the pharmaceutical preparations according to the present invention may further comprise excipients, lubricants, binders, disintegrants, isotonic agents emulsifying agents and the like.

Compound (I) or (IA), or the pharmaceutically acceptable salt thereof is usually administered systemically or locally in the oral or parenteral form when used for the above purposes. The dose and the frequency of administration may vary with the dosage form, the age and weight of patients, nature or severity of the condition to be treated, and the like. Ordinarily, it is preferable that it is administered at a dose of from 1 to 900 mg, preferably from 1 to 200 mg per day for an adult at 3 or 4 different times. However, the dose and the frequency of administration may vary with the above various conditions and the like.

### Best Mode for Carrying Out the Invention

The present invention is illustrated more specifically by referring to Examples and Reference Examples as followes. However, the scope of the present invention is not limited to these Examples.

In proton nuclear magnetic resonance spectrum (¹H-NMR) used in Reference Examples and Examples, description of multiplicity of a signal which is ordinarily used is employed, and br indicates a signal which is apparently broad.

### Reference Example 1:

### 2-(4-Bromophenyl)-6-fluoro-3-methylquinoline-4-carboxylic acid (Compound A)

5-Fluroisatin(2.33 g, 14.08 mmol) and 4-bromopropiophenone(2.00 g, 9.39 mmol) were dissolved in 24 mL of ethanol, and 24 mL of a 6 mol/L potassium hydroxide aqueous solution was added thereto. Then, the mixture was heated and refluxed for overnight. After completion of the reaction, 150 mL of water and 39 mL of diethyl ether were added to the mixture, and the mixture was extracted. The resulting aqueous layer was stirred under ice cooling, and acetic acid was slowly added to acidify the solution. Crystals precipitated were collected by filtration, and washed with 20 mL of water to obtain 3.27 g of the title compound (compound A) as a white crystal (yield 97%).
¹H NMR (δ ppm, DMSO-d₆): 8.14 (dd, J=5.7, 9.2 Hz, 1H), 7.72 (d, J=8.4 Hz, 2H), 7.78-7.68 (m, 1H), 7.58 (d, J=8.4 Hz, 2H), 7.49 (dd, J=2.7, 9.9 Hz, 1H), 3.4 (brs. 1H), 2.39 (s, 3H)
Mass (m/z): 359 (M⁻-1), 361 (M⁻+1)
IR (KBr): 1620, 1595, 1504, 1392, 1244, 1194, 1012, 991,831 cm⁻¹
m.p.: >300°C

### Reference Example 2:

### 6-Fluoro-3-methyl-2-([1,1':2',1"-terphenyl)-4-yl)quinoline-4-carboxylic acid (compound C)

Compound A (100 mg, 0.28 mmol) prepared in Reference Example 1, 2-biphenylboric acid (66 mg, 0.34 mmol) and bis(tri-o-tolylphosphine)palladium (II) dichloride (22 mg, 0.03 mmol) were dissolved in 4 mL of ethanol, and triethylamine (0.12 mL, 0.83 mmol) was added thereto. The mixture was heated and refluxed at 90°C for approximately 2 hours. After completion of the reaction, the solvent was distilled off, and to the resulting residue was added 50 mL of a 2 mol/L sodium hydroxide aqueous solution and diethyl ether to wash the residure. After the aqueous layer was filtered, the filtrate was stirred under ice cooling, and acetic acid was slowly added dropwise to the solution to neutralize. Crystals precipitated were collected by filtration, and recrystallized from a DMF-water mixed solvent to obtain 70 mg of the title compound (compound C) as a white powder (yield 58%).
¹H NMR (δ ppm, DMSO-d₆): 8.13 (dd, J=5.7, 9.2 Hz, 1H), 7.70 (dt, J=3.0, 9.2 Hz, 1H), 7.51-7.45 (m, 8H), 7.29-7.17 (m, 7H), 2.36 (s, 3H)
Mass (m/z): 434 (M+1)
IR (KBr) : 3100, 2500, 1718, 1626, 1504, 1475, 1284, 1238, 1192, 1007, 989, 829, 750, 702 cm⁻¹
m.p.: >300°C

The following Reference Examples 3 to 8 were performed in the same manner as that of Reference Example 2 using corresponding boronic acid respectively to obtain compounds D to F and H to J.

### Reference Example 3:

### 6-Fluoro-2-(2'-methoxybiphenyl-4-yl)-3-methylquinoline-4-carboxylic acid (compound D)

Yield: 70% (recrystallized from ethyl acetate; white powder)
¹H NMR (δ ppm, DMSO-d₆): 8.14 (dd, J=5.4, 9.2 Hz, 1H), 7.76-7.60 (m, 5H), 7.49 (dd, J=2.4, 9.7 Hz, 1H), 7.41-7.36 (m, 2H), 7.16 (d, J=8.1 Hz, 1H), 7.07 (t, J=8.1 Hz, 1H), 3.82 (s, 3H), 2.48 (s, 3H)
Mass (m/z): 388 (M+1)
IR (KBr): 1626, 1599, 1506, 1489, 1234, 1190, 1028, 746, 737 cm⁻¹
m.p.: 283-295°C

### Reference Example 4:

### 6-Fluoro-2-(5'-fluoro-2'-methoxybiphenyl-4-yl)-3-methylquinoline-4-carboxylic acid (compound E)

Yield: 42% (recrystallized from ethyl acetate; white powder)
¹H NMR (δ ppm, DMSO-d₆): 8.14 (dd, J=5.4, 9.2 Hz, 1H), 7.73-7.66 (m, 5H), 7.50 (dd, J=2.7, 9.7 Hz, 1H), 7.25 (dt, J=2.7, 9.2 Hz, 1H), 7.22-7.17 (m, 2H), 3.80 (s, 3H), 2.47 (s, 3H)
Mass (m/z): 406 (M+1)
IR (KBr): 1707, 1626, 1495, 1392, 1252, 1236, 1190, 1041, 721 cm⁻¹
m.p.: >300°C

### Reference Example 5

### 2-(2',4'-dimethoxybiphenyl-4-yl)-6-fluoro-3-methylquinoline-4-carboxylic acid (compound F)

Yield: 69% (recrystallized from ethyl acetate; white powder)
¹H NMR (δ ppm, DMSO-d₆): 8.13 (dd, J=5.4, 9.2 Hz, 1H), 7.72 (dt, J=2.7, 9.2 Hz, 1H), 7.65-7.57 (m, 4H), 7.48 (dd, J=2.7, 9.7 Hz, 1H), 7.32 (d, J=8.4 Hz, 1H), 6.71 (d, J=2.7 Hz, 1H), 6.65 (dd, J=2.7, 8.4 Hz, 1H), 3.83 (s, 3H), 3.82 (s, 3H), 2.38 (s, 3H)
Mass (m/z): 418 (M+1)
IR (KBr): 2931, 2839, 1709, 1701, 1608, 1579, 1498, 1302, 1282, 1246, 1236, 1205, 1030, 821 cm⁻¹
m.p.: >300°C

### Reference Example 6:

### 6-Fluoro-3-methyl-2-(2'-methylbiphenyl-4-yl)quinoline-4-carboxylic acid (compound H)

Yield: 65% (recrystallized from an ethanol-water mixed solvent; brown granular crystal)
¹H NMR (δ ppm, DMSO-d₆): 8.14 (dd, J=5.7, 9.2 Hz, 1H), 7.74-7.67 (m, 1H), 7.68 (d, J=8.4 Hz, 2H), 7.48 (d, J=8.4 Hz, 2H), 7.52-7.41 (m, 2H), 7.32-7.26 (m, 3H), 2.46 (s, 3H), 2.31 (s, 3H)
Mass (m/z): 372 (M+1)
IR (KBr) : 2990, 1714, 1709, 1504, 1485, 1387, 1236, 1192, 858, 825, 752, 716 cm⁻¹
m.p.: 260°C (decomp.)

### Reference Example 7:

### 2-(2'-Ethoxybiphenyl-4-yl)-6-fluoro-3-methylquinoline-4-carboxylic acid (compound I)

Yield: 23% (recrystallized from an ethanol-water mixed solvent; white powder)
¹H NMR (δ ppm, DMSO-d₆): 8.13 (dd, J=5.4, 9.2 Hz, 1H), 7.75-7.61 (m, 5H), 7.48 (dd, J=2.4, 9.7 Hz, 1H), 7.42-7.30 (m, 2H), 7.15-7.00 (m, 2H), 4.09 (q, J=7.0 Hz, 2H), 2.46 (s, 3H), 1.31 (t, J=7.0 Hz, 3H)
Mass (m/z): 402 (M+1)
IR (KBr): 1626, 1487, 1394, 1236, 1043, 754 cm⁻¹
m.p.: 225-227°C

### Reference Example 8:

### 6-Fluoro-2-(2'-formylbiphenyl-4-yl)-3-methylquinoline-4-carboxylic acid (compound J)

Yield: 90% (recrystallized from an ethanol-water mixed solvent; white powder)
¹H NMR (δ ppm, DMSO-d₆): 9.99 (s, 1H), 8.07 (dd, J=5.7, 9.2 Hz, 1H), 7.97 (dd, J=1.1, 9.2 Hz, 1H), 7.83-7.78 (m, 1H), 7.74 (d, J=8.4 Hz, 2H), 7.58 (d, J=8.4 Hz, 2H), 7.68-7.55 (m, 4H), 2.44 (s, 3H)
Mass (m/z): 386 (M+1)
IR (KBr): 1703, 1693, 1238, 1196, 1097, 991, 858, 769, 756, 719 cm⁻¹
m.p.: >300°C

### Reference Example 9:

### 6-Fluoro-2-(2'-hydroxymethylbiphenyl-4-yl)-3-methylquinoline-4-carboxylic acid (compound K)

Compound J (300 mg, 0.78 mmol) prepared in Reference Example 8 was dissolved in 20 mL of methanol, and sodium borohydride (12 mg, 0.31 mmol) which was dissolved in 0.5 mL of water was slowly added dropwise to the solution at room temperature. After the reaction mixture was heated and refluxed at 60°C for 2 hours, the mixture was cooled to room temperature, and 2 mL of a saturated aqueous solution of ammonium chloride was added, and then the mixture was stirred for 1 hour. After the solvent was distilled off, to the residue were added 50 mL of water and 2 mol/L of hydrochloric acid to neutralize. Crystals precipitated were collected by filtration to obtain 174 mg of the title compound (compound K) as a white powder (yield 58%).
¹H NMR (δ ppm, DMSO-d₆): 8.15 (dd, J=5.7, 9.4 Hz, 1H), 7.77-7.68 (m, 1H), 7.68 (d, J=8.1 Hz, 2H), 7.63-7.52 (m, 2H), 7.53 (d, J=8.1 Hz, 2H), 7.45-7.31 (m, 3H), 5.21 (brs, 1H), 4.48 (s, 2H), 2.48 (s, 3H)
Mass (m/z): 388 (M+1)
IR (KBr): 1620, 1504, 1356, 1296, 1248, 1196, 831 cm⁻¹
m.p.: 258-260°C

### Reference Example 10:

### 6-Fluoro-3-methyl-2-[2'-(4-phenylpiperazin-1-yl)methylbiphenyl-4-yl]quinoline-4-carboxylic acid (compound L)

Compound J (400 mg, 1.03 mmol) prepared in Reference Example 8 was dissolved in 7 mL of dichloromethane, and phenylpiperazine (0.24 mL, 1.57 mmol), sodium triacetoxyborohydride (308 mg, 1. 45 mmol) and 0.5 mL of acetic acid were added. The mixture was stirred overnight at room temperature. After completion of the reaction, 100 mL of water was added to the reaction mixture, and the solution was extracted with 200 mL of chloroform. The organic layer was dried over magnesium sulfate, and then the solvent was distilled off. The resulting residue was recrystallized from ethanol and a DMF-water mixed solvent to obtain 447 mg of the title compound (compound L) as a white powder (yield 81%).
¹H NMR (δ ppm, DMSO-d₆): 8.13 (dd, J=5.7, 9.2 Hz, 1H), 7.68 (d, J=8.1 Hz, 2H), 7.74-7.58 (m, 2H), 7.57 (d, J=8.1 Hz, 2H), 7.49 (dd, J=2.7, 10.0 Hz, 1H), 7.45-7.34 (m, 3H), 7.18 (t, J=7.3 Hz, 2H), 6.89 (d, J=7.3 Hz, 2H), 6.75 (t, J=7.3 Hz, 1H), 3.59 (s, 2H), 3.10 (t, J=4.3 Hz, 4H), 2.54 (t, J=4.3 Hz, 4H), 2.44 (s, 3H)
Mass (m/z): 532 (M+1)
IR (KBr): 1599, 1498, 1396, 1338, 1234, 1184, 837, 768 cm⁻¹
m.p.: 189-190°C

The following Reference Examples 11 and 12 were conducted in the same manner as that of Reference Example 10 using corresponding amines respectively to obtain compounds M and N.

### Reference Example 11:

### 6-Fluoro-3-methyl-2-(2'-morpholinomethylbiphenyl-4-yl)quinoline-4-carboxylic acid (compound M)

Yield: 80% (recrystallized from an ethanol-water mixed solvent; pale yellow powder)
¹H NMR (δ ppm, DMSO-d₆): 8.14 (dd, J=5.9, 9.4 Hz, 1H), 7.75-7.69 (m, 1H), 7.67 (d, J=8.1 Hz, 2H), 7.56 (d, J=8.1 Hz, 2H), 7.56-7.48 (m, 2H), 7.43-7.32 (m, 3H), 3.55 (t, J=4.3 Hz, 4H), 3.50 (s, 2H), 2.49 (s, 3H), 2.34 (t, J=4.3 Hz, 4H)
Mass (m/z): 457 (M+1)
IR (KBr): 1626, 1604, 1601, 1495, 1385, 1333, 1230, 1186, 1134, 987, 829, 750 cm⁻¹
m.p.: 163-165°C

### Reference Example 12:

### 6-Fluoro-3-methyl-2-[(2'-phenylamino)methylbiphenyl-4-yl]quinoline-4-carboxylic acid (compound N)

Yield: 64% (recrystallized from an ethanol-water mixed solvent; white powder)
¹H NMR (δ ppm, DMSO-d₆): 8.15 (dd, J=5.4, 9.2 Hz, 1H), 7.75-7.69 (m, 1H), 7.70 (d, J=8.1 Hz, 2H), 7.57 (d, J=8.1 Hz, 2H), 7.54-7.48 (m, 2H), 7.40-7.36 (m, 3H), 7.00 (t, J=8.4 Hz, 2H), 6.48 (t, J=8.4 Hz, 2H), 6.46 (d, J=8.4 Hz, 1H), 4.21 (s, 2H), 2.45 (s, 3H)
Mass (m/z): 463 (M+1)
IR (KBr) : 1716, 1624, 1616, 1602, 1558, 1508, 1238, 1191, 750 cm⁻¹
m.p.: 222-224°C

### Reference Example 13:

### 6-Fluoro-3-methyl-2-([1,1':2',1"-terphenyl]-4-yl)quinone-4-carboxamide (compound O)

Compound C (615 mg, 1.42 mmol) prepared in Reference Example 2 was slowly added to 6 mL of ice-cooled thionyl chloride, and the mixture was stirred at room temperature for 2 hours. After completion of the reaction, thionyl chloride was distilled off, and to the residue was added 50 mL of toluene, and the solvent was distilled off again. To the resulting residue were added 20 mL of THF, and 5 mL of aqueous ammonia was slowly added under ice cooling, and then the mixture was stirred at room temperature for 30 minutes. After completion of the reaction, the solvent was distilled off. The resulting residue was purified by silica gel column chromatography (chloroform/methanol/aqueous ammonia = 100/10/1), and then recrystallized from an ethanol-water mixed solvent to obtain 270 mg (0.625 mmol) of the title compound (compound O) as a pale brown crystal (yield 44%).
¹H NMR (δ ppm, CDCl₃): 8.11 (dd, J=5.3, 9.2 Hz, 1H), 7.55-7.41 (m, 7H), 7.39 (d, J=8.3 Hz, 2H), 7.28 (d, J=8.3 Hz, 2H), 7.26-7.24 (m, 4H), 6.15 (brs, 1H), 5.90 (brs, 1H), 2.44 (s, 3H)
Mass (m/z): 433 (M+1)
IR (KBr): 1684, 1668, 1626, 1497, 1398, 1234, 831, 748 cm⁻¹
m.p.: 243-246°C

### Reference Example 14:

### 4-Amino-6-fluoro-3-methyl-2-([1,1':2',1"-terphenyl]-4-yl)quinoline (compound P)

Bromine (0.046 mL, 0.90 mmol) and compound O (300 mg, 0.69 mmol) prepared in Reference Example 13 were added to 1.6 mL of an ice-cooled 17% potassium hydroxide aqueous solution in the order. After homogenizing, the solution was stirred at 70°C for 1 hour. After completion of the reaction, acetic acid was added under ice cooling to acidify the solution. Subsequently, the solution was neutralized with a 2 mol/L sodium hydroxide aqueous solution. The resulting solution was extracted with ethyl acetate. The organic layer was washed with 100 mL of brine and with 100 mL of water, and dried over magnesium sulfate. The solvent was distilled off, and the resulting residue was recrystallized from an ethanol-water mixed solvent to obtain 117 mg (0.29 mmol) of the title compound (compound P) as a white crystal (yield 42%).
¹H NMR (δ ppm, CDCl₃): 8.03 (dd, J=5.4, 9.2 Hz, 1H), 7.49-7.32 (m, 5H), 7.38 (d, J=8.4 Hz, 2H), 7.27-7.19 (m, 6H), 7.25 (d, J=8.4 Hz, 2H), 4.62 (brs, 2H), 2.17 (s, 3H)
Mass (m/z): 405 (M+1)
IR (KBr): 1641, 1633, 1502, 1377, 1203, 1009, 850, 833, 748 cm⁻¹
m.p.: 227-229°C

The following Examples 1 to 9 were conducted in the same manner as that of Reference Example 2 using compound B prepared in Example 21 which is described later and corresponding boronic acid respectively to obtain compounds 1 to 9.

### Example 1:

### 6-Fluoro-4-hydroxymethyl-3-methyl-2-([1,1':2',1"-terphenyl]-4-yl)quinoline (compound 1)

Yield: 48% (recrystallized from an ethanol-water mixed solvent: white crystal)
¹H NMR (δ ppm, DMSO-d₆): 8.04 (dd, J=5.7, 9.2 Hz, 1H), 7.96 (dd, J=2.7, 11.1 Hz, 1H), 7.61 (dt, J=2.7, 8.4 Hz, 1H), 7.51 (d, J=3.0 Hz, 2H), 7.51-7.40 (m, 2H), 7.40 (d, J=8.4 Hz, 2H), 7.25 (d, J=3.0 Hz, 2H), 7.24 (d, J=8.4 Hz, 2H), 7.32-7.17 (m, 3H), 5.35 (t, J=5.7 Hz, 1H), 4.94 (d, J=5.7 Hz, 2H), 2.42 (s, 3H)
Mass (m/z): 420 (M+1)
IR (KBr): 3156, 1626, 1500, 1473, 1354, 1190, 1004, 831, 748, 702 cm⁻¹
m.p.: 121-123°C

### Example 2:

### 6-Fluoro-4-hydroxymethyl-2-(2'-methoxybiphenyl-4-yl)-3-methylquinoline (compound 2)

Yield: 42% (recrystallized from an ethanol-water mixed solvent: white powder)
¹H NMR (δ ppm, DMSO-d₆): 8.04 (dd, J=5.9, 9.4 Hz, 1H), 7.99 (dd, J=3.0, 11.3 Hz, 1H), 7.66-7.57 (m, 5H), 7.41-7.35 (m, 2H), 7.17-7.05 (m, 2H), 5.38 (t, J=5.4 Hz, 1H), 4.98 (d, J=5.4 Hz, 2H), 3.82 (s, 3H), 2.92 (s, 3H)
Mass (m/z): 374 (M+1)
IR (KBr): 1502, 1495, 1238, 1190, 1005, 829, 750 cm⁻¹
m.p.: 178-179°C

### Example 3:

### 2-(2'-Ethoxybiphenyl-4-yl)-6-fluoro-4-hydroxymethyl-3-methylquinoline (compound 3)

Yield: 45% (recrystallized from ethanol: white powder)
¹H NMR (δ ppm, DMSO-d₆): 8.05 (dd, J=5.9, 9.4 Hz, 1H), 7.99 (dd, J=2.7, 11.1 Hz, 1H), 7.66 (d, J=8.1 Hz, 2H), 7.67-7.57 (m, 1H), 7.58 (d, J=8.1 Hz, 2H), 7.42-7.32 (m, 2H), 7.15-7.03 (m, 2H), 5.39 (t, J=5.4 Hz, 1H), 4.97 (d, J=5.4 Hz, 2H), 4.09 (q, J=7.0 Hz, 2H), 2.44 (s, 3H), 1.31 (t, J=7.0 Hz, 3H)
Mass (m/z): 388 (M+1)
IR (KBr): 1502, 1487, 1435, 1236, 1192, 1045, 1005, 750 cm⁻¹
m.p.: 140-143°C

### Example 4:

### 2-(3'-Ethoxybiphenyl-4-yl)-6-fluoro-4-hydroxymethyl-3-methylquinoline (compound 4)

Yield: 51% (recrystallized from ethanol: white powder)
¹H NMR (δ ppm, DMSO-d₆): 8.05 (dd, J=5.9, 9.2 Hz, 1H), 7.99 (dd, J=2.7, 11.3 Hz, 1H), 7.80 (d, J=8.6 Hz, 2H), 7.64 (d, J=8.6 Hz, 2H), 7.41 (t, J=8.4 Hz, 1H), 7.32-7.26 (m, 3H), 6.95 (dd, J=1.6, 8.4 Hz, 1H), 5.39 (t, J=5.4 Hz, 1H), 4.98 (d, J=5.4 Hz, 2H), 4.13 (q, J=7.0 Hz, 2H), 2.50 (s, 3H), 1.37 (t, J=7.0 Hz, 3H)
Mass (m/z): 388 (M+1)
IR (KBr): 1604, 1581, 1504, 1485, 1300, 1209, 1190, 1053, 850, 843, 781 cm⁻¹
m.p.: 199-200°C

### Example 5:

### 2-(4'-Ethoxybiphenyl-4-yl)-6-fluoro-4-hydroxymethyl-3-methylquinoline (compound 5)

Yield: 70% (recrystallized from ethanol: white powder)
¹H NMR (δ ppm, DMSO-d₆): 8.05 (dd, J=5.9, 7.2 Hz, 1H), 7.99 (dd, J=3.0, 11.1 Hz, 1H), 7.75 (d, J=8.1 Hz, 2H), 7.71-7.65 (m, 1H), 7.69 (d, J=8.6 Hz, 2H), 7.60 (d, J=8.1 Hz, 2H), 7.05 (d, J=8.6 Hz, 2H), 5.38 (t, J=5.4 Hz, 1H), 4.97 (d, J=5.4 Hz, 2H), 4.09 (q, J=7.0 Hz, 2H), 2.51 (s, 3H), 1.37 (t, J=7.0 Hz, 3H)
Mass (m/z): 388 (M+1)
IR (KBr): 1604, 1498, 1481, 1354, 1248, 1201, 1047, 829 cm⁻¹
m.p.: 211-212°C

### Example 6:

### 6-Fluoro-2-(2'-fluorobiphenyl-4-yl)-4-hydroxymethyl-3-methylquinoline (compound 6)

Yield: 90% (recrystallized from ethanol: pale brown powder)
¹H NMR (δ ppm, DMSO-d₆): 8.05 (dd, J=5.9, 9.2 Hz, 1H), 7.99 (dd, J=3.0, 11.3 Hz, 1H), 7.73-7.63 (m, 4H), 7.51-7.42 (m, 1H), 7.40-7.31 (m, 4H), 5.40 (t, J=5.4 Hz, 1H), 4.98 (d, J=5.4 Hz, 2H), 2.52 (s, 3H)
Mass (m/z): 362 (M+1)
IR (KBr): 1485, 1238, 1194, 1007, 827, 748 cm⁻¹
m.p.: 202-204°C

### Example 7:

### 6-Fluoro-2-(5'-fluoro-2'-methoxybiphenyl-4-yl)-4-hydroxymethyl-3-methylquinoline (compound 7)

Yield: 72% (recrystallized from ethanol: pale brown powder)
¹H NMR (δ ppm, DMSO-d₆): 8.04 (dd, J=5.7, 9.2 Hz, 1H), 7.98 (dd, J=2.7, 11.1 Hz, 1H), 7.64 (d, J=7.6 Hz, 2H), 7.63-7.58 (m, 1H), 7.59 (d, J=8.4 Hz, 2H), 7.28-7.13 (m, 3H), 5.37 (t, J=5.4 Hz, 1H), 4.98 (d, J=5.4 Hz, 2H), 3.80 (s, 3H), 2.52 (s, 3H)
Mass (m/z): 392 (M+1)
IR (KBr): 1495, 1255, 1236, 1178, 1043, 1036, 1005, 843, 829 cm⁻¹
m.p.: 202-203°C

### Example 8

### 2-(2',4'-dimethoxybiphenyl-4-yl)-6-fluoro-4-hydroxymethyl-3-methylquinoline (compound 8)

Yield: 55% (recrystallized from ethanol: white powder)
¹H NMR (δ ppm, DMSO-d₆): 8.05 (dd, J=5.7, 8.9 Hz, 1H), 7.98 (dd, J=2.7, 11.3 Hz, 1H), 7.66-7.50 (m, 5H), 7.31 (d, J=8.4 Hz, 1H), 6.70 (d, J=2.4 Hz, 1H), 6.65 (dd, J=2.4, 8.4 Hz, 1H), 5.38 (t, J=5.4 Hz, 1H), 4.97 (d, J=5.4 Hz, 2H), 3.83 (s, 3H), 3.81 (s, 3H), 2.52 (s, 3H)
Mass (m/z): 404 (M⁺)
IR (KBr): 1608, 1497, 1238, 1209, 1162, 1053, 1030, 1005, 833 cm⁻¹
m.p.: 205-207°C

### Example 9

### 6-Fluoro-2-(2'-formylbiphenyl-4-yl)-4-hydroxymethyl-3-methylquinoline (compound 9)

Yield: 95% (recrystallized from ethanol: pale brown powder)
¹H NMR (δ ppm, DMSO-d₆): 10.00 (s, 1H), 8.10-7.95 (m, 3H), 7.81 (dd, J=6.2, 7.3 Hz, 1H), 7.66-7.63 (m, 3H), 7.65 (m, 4H), 5.41 (t, J=5.4 Hz, 1H), 5.00 (d, J=5.4 Hz, 2H), 2.54 (s, 3H)
Mass (m/z): 372 (M+1)
IR (KBr): 1693, 1597, 1504, 1446, 1236, 1194, 1005, 829, 750 cm⁻¹
m.p.: 192-194°C

In the same manner as that of Reference Example 10, compound 10, 11 and 13, and free bases of compound 12 and 14 were obtained from compound 9 prepared in Example 9 and corresponding amine respectively.

### Example 10:

### 6-Fluoro-4-hydroxymethyl-3-methyl-2-[2'-(methylamino)methylbiphenyl-4-yl]quinoline (compound 10)

Yield: 11% (recrystallized from ethanol: pale brown powder)
¹H NMR (δ ppm, DMSO-d₆): 8.07 (dd, J=5.9, 9.1 Hz, 1H), 7.99 (dd, J=2.7, 11.3 Hz, 1H), 7.63 (d, J=8.4 Hz, 2H), 7.54 (d, J=8.4 Hz, 2H), 7.69-7.53 (m, 2H), 7.44-7.31 (m, 3H), 5.41 (t, J=4.6 Hz, 1H), 4.98 (d, J=4.6 Hz, 2H), 3.71 (s, 2H), 2.51 (s, 3H), 2.29 (s, 3H)
Mass (m/z): 387 (M+1)
IR (KBr): 1610, 1336, 1250, 1199, 1024, 1005 cm⁻¹
m.p.: 193-195°C

### Example 11:

### 6-Fluoro-4-hydroxymethyl-3-methyl-2-[2'-(4-phenylpiperazin-1-yl)methylbiphenyl-4-yl]quinoline (compound 11)

Yield: 52% (recrystallized from ethanol: white powder)
¹H NMR (δ ppm, DMSO-d₆): 8.06 (dd, J=5.9, 9.2 Hz, 1H), 7.99 (dd, J=2.7, 10.8 Hz, 1H), 7.67-7.56 (m, 5H), 7.44-7.30 (m, 3H), 7.19 (d, J=8.4 Hz, 2H), 7.18 (t, J=8.4 Hz, 1H), 6.89 (d, J=8.4 Hz, 2H), 6.75 (d, J=7.3 Hz, 1H), 5.38 (d, J=5.4 Hz, 1H), 4.98 (d, J=5.4 Hz, 2H), 3.52 (s, 2H), 3.09 (t, J=4.3 Hz, 4H), 2.49 (s, 3H), 2.47 (t, J=4.3 Hz, 4H)
Mass (m/z): 518 (M+1)
IR (KBr) : 1599, 1504, 1497, 1452, 1352, 1228, 1189, 1007, 831, 764 cm⁻¹
m.p.: 154-156°C

### Example 12:

### 6-Fluoro-4-hydroxymethyl-3-methyl-2-(2'-morpholinomethylbiphenyl-4-yl)quinoline dihydrochloride (compound 12)

A 4 mol/L hydrochloric acid-ethyl acetate solution (338 µL, 1.35 mmol) was added to a free base of compound 12 prepared in a manner as that of Reference Example 10, and the solvent was distilled off. Then, the residue was recrystallized from ethanol to obtain 91 mg (0.176 mmol) of the title compound (compound 12) as a pale brown crystal (yield 65%).
¹H NMR (as a free base) (δ ppm, DMSO-d₆): 8.08 (dd, J=5.9, 9.2 Hz, 1H), 8.00 (dd, J=2.6, 11.1 Hz, 1H), 7.67-7.54 (m, 6H), 7.40-7.33 (m, 3H), 5.39 (t, J=4.6 Hz, 1H), 4.98 (d, J=4.6 Hz, 2H), 3.54 (t, J=4.3 Hz, 4H), 3.45 (s, 2H), 2.52 (s, 3H), 2.30 (t, J=4.3 Hz, 4H)
Mass (m/z): 443 (M⁺)
IR (KBr): 1614, 1261, 1250, 1124, 1024, 1018, 1007, 858, 849, 767 cm⁻¹
m.p.: 190-192°C

### Example 13:

### 6-Fluoro-4-hydroxymethyl-3-methyl-2-[2'-(phenylamino)methylbiphenyl-4-yl]quinoline (compound 13)

Yield: 37% (recrystallized from ethanol: white powder)
¹H NMR (δ ppm, DMSO-d₆): 8.06 (dd, J=5.9, 9.2 Hz, 1H), 7.99 (dd, J=2.4, 11.3 Hz, 1H), 7.63 (d, J=8.6 Hz, 2H), 7.60-7.51 (m, 2H), 7.57 (d, J=8.6 Hz, 2H), 7.40-7.34 (m, 3H), 7.00 (t, J=7.6 Hz, 2H), 6.51-6.48 (m, 1H), 6.48 (t, J=8.6 Hz, 2H), 6.15 (t, J=4.1 Hz, 1H), 5.39 (t, J=4.6 Hz, 1H), 4.98 (t, J=4.6 Hz, 2H), 4.21 (d, J=4.1 Hz, 2H), 2.51 (s, 3H)
Mass (m/z): 449 (M+1)
IR (KBr): 1601, 1508, 1504, 1477, 1361, 1275, 1196, 1014, 1005, 837, 746 cm⁻¹
m.p.: 201-202°C

### Example 14:

### 6-Fluoro-2-{2'-[(2-hydroxyethyl)amino]methylbiphenyl-4-yl}-4-hydroxymethyl-3-methylquinoline·dihydrochloride (compound 14)

In a manner similar to that in Example 12, compound 14 was obtained from free base of compound 14 prepared in a manner similar to that in Example 10.
Yield 37% (recrystallized from ethanol: white powder)
¹H NMR (as a free base) (δ ppm, DMSO-d₆): 8.07 (dd, J=5.9, 9.1 Hz, 1H), 7.99 (dd, J=2.7, 11.3 Hz, 1H), 7.67-7.50 (m, 2H), 7.58 (m, 4H), 7.42-7.29 (m, 3H), 4.99 (s, 2H), 3.70 (s, 2H), 3.42 (t, J=5.9 Hz, 2H), 2.54 (t, J=5.9 Hz, 2H), 2.51 (s, 3H)
Mass (m/z): 417 (M+1)
IR (KBr): 2340, 1608, 1578, 1508, 1458, 1336, 1252, 1203 cm⁻¹
m.p.: 203-205°C

### Example 15:

### Methyl 6-fluoro-3-methyl-2-([1,1':2',1"-terphenyl]-4-yl)quinoline-4-carboxylate (compound 15)

Compound C (420 mg, 0.97 mmol) prepared in Reference Example 1 was slowly added to 6 mL of ice-cooled thionyl chloride, and the mixture was stirred at room temperature for 2 hours. After completion of the reaction, thionyl chloride was distilled off, to the residue was added 50 mL of toluene, and the solvent was distilled off again. To the resulting residue was added 20 mL of THF, and 5 mL of methanol was slowly added under ice cooling, then the mixture was stirred at room temperature for 30 minutes. After completion of the reaction, the solvent was distilled off, and the resulting residue was purified by silica gel column chromatography (hexane/ethyl acetate = 10/1), and then recrystallized from a methanol-water mixed solvent to obtain 136 mg (0.304 mmol) of the title compound (compound 15) as a pale brown crystal (yield 31%).
¹H NMR (δ ppm, DMSO-d₆): 8.15 (dd, J=5.4, 9.2 Hz, 1H), 7.72 (dd, J=2.7, 9.2 Hz, 1H), 7.53-7.43 (m, 7H), 7.29-7.16 (m, 7H), 4.06 (s, 3H), 2.33 (s. 3H)
Mass (m/z): 448 (M+1)
IR (KBr): 1734, 1699, 1653, 1558, 1540, 1522, 1508, 1473, 1458, 1223, 750 cm⁻¹
m.p.: 164-165°C

### Example 16:

### 4-Acetyl-6-fluoro-3-methyl-2-([1,1':2',1"-terphenyl]-4-yl)quinoline (compound 16)

Compound 15 (136 mg, 0.304 mmol) prepared in Example 15 was dissolved in 10 mL of THF under an argon atmosphere, and the solution was cooled to -78°C. Then, a diethyl ether solution of 1.03 mol/L methyllithium (0.89 mL, 0.91 mmol) was slowly added. After completion of the reaction, to the mixture was added 2 mL of a saturated aqueous solution of ammonium chloride, and the mixture was warmed to room temperature. Then, to the mixture was added 100 mL of water, and the mixture was extracted with ethyl acetate. The solvent was distilled off, and the resulting residue was recrystallized from ethanol to obtain 85 mg (0.198 mmol) of the title compound (compound 16) as a pale brown crystal (yield 65%).
¹H NMR (δ ppm, DMSO-d₆): 8.13 (dd, J=5.7, 9.2 Hz, 1H), 7.70 (dt, J=3.0, 9.2 Hz, 1H), 7.49 (d, J=7.7 Hz, 2H), 7.51-7.38 (m, 5H), 7.25 (d, J=7.7 Hz, 2H), 7.29-7.16 (m, 5H), 2.69 (s, 3H), 2.27 (s, 3H)
Mass (m/z): 432 (M+1)
IR (KBr) : 1708, 1234, 1200, 1007, 831, 779, 766, 748 cm⁻¹
m.p.: 75-77°C

### Example 17:

### 6-Fluoro-3-methyl-2-([1,1':2',1"-terphenyl]-4-yl)quinoline-4-carbonitrile (compound 17)

Compound O (300 mg, 0.65 mmol) prepared in Reference Example 13 was added to 10 mL of phosphoryl chloride, and the mixture was heated and refluxed for 2 hours. After completion of the reaction, phosphoryl chloride was distilled off, and to the mixture was added 50 mL of toluene, then the solvent was distilled off again. To the resulting residue was added 100 mL of water and a saturated aqueous solution of sodium hydrogencarbonate to neutralize the solution. Then, the solution was extracted with chloroform. The solvent was distilled off from the organic layer. The resulting residue was purified by silica gel column chromatography (hexane/ethyl acetate = 5/1), and then recrystallized from an ethanol-water mixed solvent to obtain 95 mg (0.23 mmol) of the title compound (compound 17) as a pale brown crystal (yield 35%).
¹H NMR (δ ppm, DMSO-d₆): 8.25 (dd, J=5.3, 9.2 Hz, 1H), 7.82 (dt, J=3.0, 8.9 Hz, 1H), 7.75 (dd, J=2.6, 8.9 Hz, 1H), 7.54 (d, J=8.3 Hz, 2H), 7.55-7.47 (m, 4H), 7.29-7.24 (m, 3H), 7.23 (d, J=8.3 Hz, 2H), 7.20-7.19 (m, 2H), 2.63 (s, 3H)
Mass (m/z): 415 (M+1)
IR (KBr): 2230, 1238, 1194, 989, 835, 766 cm⁻¹
m.p.: 153-155°C

### Example 18:

### 3,4-Dimethyl-6-fluoro-2-([1,1':2',1"-terphenyl]-4-yl)quinoline (compound 18)

Compound 1 (250 mg, 0.60 mmol) prepared in Example 1 was slowly added to 10 mL of ice-cooled thionyl chloride, and the mixture was stirred at room temperature for 2 hours. After completion of the reaction, thionyl chloride was distilled off, and to the residue was added 50 mL of toluene, then the solvent was distilled off again. To the resulting residue were added 5 mL of DMF and sodium borohydride (30 mg, 0.72 mmol) slowly at room temperature, then the mixture was stirred at room temperature for 30 minutes. After completion of the reaction, to the mixture was added 2 mol/L hydrochloric acid to acidify the solution. After to the solution was further added 100 mL of water, the solution was extracted with ethyl acetate. The organic layer was washed with 100 mL of brine and with 100 mL of water, and dried over magnesium sulfate. The solvent was distilled off, and the resulting residue was purified by silica gel column chromatography (hexane/ethyl acetate = 10/1), and then recrystallized from an ethanol-water mixed solvent to obtain 152 mg (0.38 mmol) of the title compound (compound 18) as a white crystal (yield 63%).
¹H NMR (δ ppm, DMSO-d₆): 8.09 (dd, J=5.6, 9.2 Hz, 1H), 7.61 (dd, J=2.6, 10.5 Hz, 1H), 7.38 (d, J=8.2 Hz, 2H), 7.51-7.37 (m, 4H), 7.26 (d, J=8.2 Hz, 2H), 7.28-7.20 (m, 6H), 2.62 (s, 3H), 2.35 (s, 3H)
Mass (m/z): 404 (M+1)
IR (KBr): 1498, 1473, 1234, 1188, 1005, 746 cm⁻¹
m.p.: 194-195°C

### Example 19:

### Methyl 6-Fluoro-2-[4-(2-methoxyphenyl)piperazin-1-yl]-3-methylquinoline-4-carboxylate (compound 19)

Compound G (330 mg, 1. 40 mmol) synthesized by the method described in WO 97/00074 and pyridine (125 µL, 1.54 mmol) were added to 10 mL of ice-cooled dichloromethane, and 264 µL (1.54 mmol) of trifluoromethanesulfonic anhydride was further added slowly. The reaction mixture was warmed to room temperature, and stirred for 1 hour. After completion of the reaction, to the reaction solution was added 100 mL of water, and the mixture was extracted with 200 mL of dichloromethane. The organic layer was washed with 100 mL of brine and with 100 mL of water, and dried over magnesium sulfate. The solvent was distilled off, and to the resulting residue was added 45 mL of acetonitrile, then 2-methoxyphenylpiperazine (296 mg, 1.54 mmol) and triethylamine (390 mL, 2.80 mmol) were added in the order, and the mixture was heated and refluxed for 2 hours. After completion of the reaction, the solvent was distilled off, and to the residue were added 100 mL of dichloromethane and 100 mL of water, and the mixture was extracted with dichloromethane. The organic layer was washed with 100 mL of brine and with 100 mL of water, and dried over magnesium sulfate. The solvent was distilled off. The resulting residue was purified by silica gel column chromatography (hexane/ethyl acetate = 10/1), and then recrystallized from an ethanol-water mixed solvent to obtain 206 mg (0.50 mmol) of the title compound (compound 19) as a white crystal (yield 36%).
¹H NMR (δ ppm, CDCl₃): 7.86 (dd, J=5.4, 9.2 Hz, 1H), 7.35 (dt. J=2.7, 8.4 Hz, 1H), 7.23 (dd, J=2.7, 10.0 Hz, 2H), 7.06-6.89 (m, 4H), 4.06 (s, 3H), 3.90 (s, 3H), 3.48 (t, J=4.3 Hz, 4H), 3.28 (t, J=4.3 Hz, 4H), 2.44 (s. 3H)
Mass (m/z): 410 (M+1)
IR (KBr): 2831, 2821, 1736, 1593, 1566, 1504, 1454, 1435, 1412, 1369, 1261, 1155, 1142, 1036, 1022, 1011, 752 cm⁻¹
m.p.: 128-129°C

### Example 20:

### 6-Fluoro-4-hydroxymethyl-2-[4-(2-methoxyphenyl)piperazin-1-yl]-3-methylquinoline (compound 20)

LAH (122 mg, 3.21 mmol) was added to 20 mL of ice-cooled THF, and compound 19 (580 mg, 1.40 mmol) prepared in Example 19 was slowly added, then the mixture was warmed to room temperature, and stirred for 1 hour. After completion of the reaction, the reaction solution was ice-cooled, and 122 µL of water, 122 µL of a 15% sodium hydroxide aqueous solution and 366 µL of water were added in the order. Then, the mixture was warmed to room temperature, and stirred for 30 minutes. After crystals precipitated were filtered-off, to the filtrate was added 100 mL of water, and the mixture was extracted with 200 mL of dichloromethane. The organic layer was washed with 100 mL of brine and with 100 mL of water, and dried over magnesium sulfate. The solvent was distilled off, and the resulting residue was purified by silica gel column chromatography (hexane/ethyl acetate = 10/1), and then recrystallized from an ethanol-water mixed solvent to obtain 126 mg (0.33 mmol) of the title compound (compound 20) as a white crystal (yield 24%).
¹H NMR (δ ppm, CDCl₃): 7.86 (dd, J=5.3, 9.2 Hz, 1H), 7.66 (dd, J=2.6, 10.8 Hz, 1H), 7.33 (dt, J=2.6, 9.2 Hz, 2H), 7.05-6.89 (m, 4H), 5.07 (d, J=5.4 Hz, 2H), 3.90 (s, 3H), 3.45 (t, J=4.0 Hz, 4H), 3.28 (t, J=4.0 Hz, 4H), 2.55 (s, 3H)
Mass (m/z): 382 (M+1)
IR (KBr): 1500, 1408, 1240, 1223, 1028, 1016, 833, 754 cm⁻¹
m.p.: 190-191°C

### Example 21:

### 2-(4-Bromophenyl)-6-fluoro-4-hydroxymethyl-3-methylquinoline (compound 21)

A mixed solution of 5 mL of DMF and 9 mL of dichloromethane was cooled to -20°C under an argon atmosphere, and a solution of oxalyl chloride (1.92 mL, 20.73 mmol) in 6 mL of dichloromethane was slowly added using a dropping funnel. After 20 minutes, a solution which was dissolving of compound A (3.39 g, 9.42 mmol) prepared in Reference Example 1 in 5 mL of DMF and N-methylmorpholine (2.07 mL, 18.84 mmol) were slowly added to the reaction mixture using a dropping funnel in the order. After the reaction mixture was stirred at -20°C for 20 minutes, hydroxybenzotriazole monohydrate (2.54 g, 18.81 mmol) and N-methylmorpholine (2.07 mL, 18.84 mmol) were added, then the mixture was warmed to room temperature, and stirred for 2 hours. Crystals precipitated were collected, washed with 20 mL of water, and then suspended in 200 mL of isopropyl alcohol. After the suspension was heated to 50°C, a solution which was dissolving sodium borohydride (1.24 g, 33.04 mmol) in 2 mL of water was slowly added dropwise. The mixture was stirred at 50°C for 1 hour, and then allowed to cool to room temperature with stirring. Crystals precipitated were collected by filtration to obtain 1.52 g (4.39 mmol) of the title compound (compound 12) as a white crystal (yield 47%).
¹H NMR (δ ppm, DMSO-d₆): 8.03 (dd, J=5.7, 9.2 Hz, 1H), 7.97 (dd, J=2.7, 11.1 Hz, 1H), 7.71 (d, J=9.2 Hz, 2H), 7.62 (dt, J=2.7, 9.2 Hz, 1H), 7.51 (d, J=9.2 Hz, 2H), 5.38 (t, J=5.4 Hz, 1H), 4.95 (d, J=5.4 Hz, 2H), 2.44 (s, 3H)
Mass (m/z): 347 (M⁺+2), 345 (M⁺)
IR (KBr): 1626, 1489, 1361, 1238, 1193, 1003, 831 cm⁻¹
m.p.: 222-240°C

### Preparation Example 1: Tablets

Tablets comprising the following composition are prepared in a conventional manner.

| | | |
|---|---|---|
| Recipe | compound 12 | 20 mg |
| | lactose | 143.4 mg |
| | potato starch | 30 mg |
| | hydroxypropyl cellulose | 6 mg |
| | magnesium stearate | 0.6 mg |
| | | 200 mg |

### Preparation Example 2: Capsules

Capsules comprising the following composition are prepared in a conventional manner.

| | | |
|---|---|---|
| Recipe | compound 1 | 20 mg |
| | Avicel | 99.5 mg |
| | magnesium stearate | 0.5 mg |
| | | 120 mg |

### Preparation Example 3: Injection

An injection comprising the following composition is prepared in a conventional manner.

| | | |
|---|---|---|
| Recipe | compound 19 | 2 mg |
| | purified soybean oil | 200 mg |
| | purified yolk lecithin | 24 mg |
| | glycerin for injection | 50 mg |
| | distilled water for injection | 1.72 mL |
| | | 2.00 mL |

### Industrial Applicability

The present invention provides a PDE10A inhibitor which has PDE10A inhibitory activity and comprises a quinoline derivative or a pharmaceutically acceptable salt thereof as an active ingredient which is useful for treating and/or preventing various diseases caused by enhancing the activity of PDE10A function (for example, a neural disease such as Parkinson' s disease, Huntington disease or Alzheimer disease, dyskinesia, hypogonadism, diabetes, an ischemic heart disease, hypertension, an inflammatory disease, a disease of the digestive system, an allergic disease, osteoporosis, pain, a malignant tumor or the like). Further it provides a quinoline derivative or a pharmaceutically acceptable salt thereof which is useful for treating and/or preventing various diseases caused by enhancing the activity of PDE10A function.

### "Sequence Listing Free Text"

SEQ ID NO. 1 - Description of Artificial Sequence: Synthetic DNA
SEQ ID NO. 2 - Description of Artificial Sequence: Synthetic DNA
SEQ ID NO. 3 - Description of Artificial Sequence: Synthetic DNA
SEQ ID NO. 4 - Description of Artificial Sequence: Synthetic DNA

## Claims

1. A phosphodiesterase 10A (PDE10A) inhibitor which comprises a quinoline derivative represented by general formula (I) 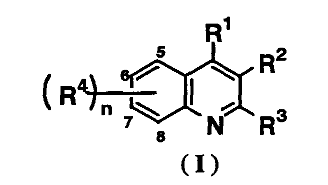 [wherein n represents an integer of from 1 to 4, R¹ represents substituted or unsubstituted lower alkyl, -C(=Y)R⁹ (wherein Y represents an oxygen atom or a sulfur atom, and R⁹ represents a hydrogen atom, hydroxy, substituted or unsubstituted lower alkyl, substituted or unsubstituted lower alkoxy, substituted or unsubstituted aryl, a substituted or unsubstituted heterocyclic group, amino, mono-lower alkylamino or di-lower alkylamino), hydroxy, halogen, cyano, amino, mono-lower alkylamino or di-lower alkyl amino, R² represents a hydrogen atom, amino, nitro, substituted or unsubstituted lower alkyl, substituted or unsubstituted lower alkoxy, -S(O)ₘR¹² (wherein R¹² represents substituted or unsubstituted lower alkyl or substituted or unsubstituted aryl, and m represents an integer of from 0 to 2), mono-lower alkylamino or di-lower alkylamino, R³ represents a hydrogen atom, halogen, hydroxy, substituted or unsubstituted lower alkyl, substituted or unsubstituted cycloalkyl, substituted or unsubstituted aryl or a substituted or unsubstituted heterocyclic group, or R² and R³ form a substituted or unsubstituted condensed ring together with two carbon atoms on roots thereof, and R⁴ represents a hydrogen atom, halogen, cyano, amino, nitro, substituted or unsubstituted lower alkyl, substituted or unsubstituted cycloalkyl, substituted or unsubstituted lower alkoxy, -S(O)ₘₐR^{12a} (wherein R^{12a} and ma have the same meanings as those of the above R¹² and m respectively), -C(=Y¹)R^{9a} (wherein Y¹ and R^{9a} have the same meanings as those of the above Y and R⁹ respectively), mono-lower alkylamino or di-lower alkylamino, and when n is an integer of 2 or more, R⁴s each may be the same or different],
or a pharmaceutically acceptable salt thereof as an active ingredient.

2. The PDE10A inhibitor according to claim 1, wherein R¹ is substituted or unsubstituted lower alkyl, -C(=Y)R⁹ (wherein Y and R⁹ have the same meanings as those above-mentioned respectively), cyano or amino, and R² is substituted or unsubstituted lower alkyl.

3. The PDE10A inhibitor according to claim 1, wherein R¹ is methyl, hydroxymethyl, acetyl, carboxy, methoxycarbonyl, cyano or amino.

4. The PDE10A inhibitor according to any one of claims 1 to 3, wherein R³ is substituted or unsubstituted aryl or a substituted or unsubstituted heterocyclic group.

5. The PDE10A inhibitor according to any one of claims 1 to 3, wherein R³ is substituted or unsubstituted biphenylyl or substituted or unsubstituted piperazinyl.

6. The PDE10A inhibitor according to any one of claims 1 to 3, wherein R³ is substituted or unsubstituted biphenyl-4-yl or substituted or unsubstituted piperazin-1-yl.

7. The PDE10A inhibitor according to any one of claims 1 to 3, wherein R³ is general formula (A) 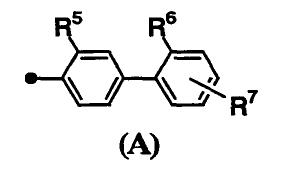 [wherein R⁵, R⁶ and R⁷, which may be the same or different, each represent a hydrogen atom, halogen, substituted or unsubstituted lower alkyl, substituted or unsubstituted lower alkoxy, aryl, substituted or unsubstituted lower alkanoyl or a substituted or unsubstituted heterocyclic group]
or piperazin-1-yl having substituted or unsubstituted lower alkyl or substituted or unsubstituted aryl as a substituent on the 4-position.

8. The PDE10A inhibitor according to any one of claims 1 to 7, wherein n is 1, and R⁴ is halogen.

9. A quinoline derivative represented by general formula (IA) 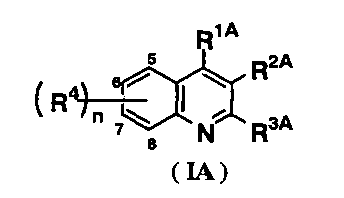 [wherein n and R⁴ have the same meanings as those above-mentioned respectively, R^{1A} represents lower alkyl, hydroxy lower alkyl, -C(=Y)R^{9A} (wherein Y has the same meaning as that above-mentioned, and R^{9A} represents a hydrogen atom, lower alkyl, lower alkoxy, amino, mono-lower alkylamino or di-lower alkylamino), cyano, amino, mono-lower alkylamino or di-lower alkylamino, R^{2A} represents amino, nitro, substituted or unsubstituted lower alkyl, substituted or unsubstituted lower alkoxy, -S (O)ₘR¹² (wherein R¹² and m have the same meanings as those above-mentioned respectively), mono-lower alkylamino or di-lower alkylamino, and R^{3A} represents a substituted or unsubstituted heterocyclic group or substituted or unsubstituted aryl, or R^{2A} and R^{3A} form cycloalkane condensed with a substituted or unsubstituted benzene ring together with two carbon atoms on roots thereof, provided that when R^{1A} is hydroxymethyl or -C(=O)R^{9B} (wherein R^{9B} represents a hydrogen atom, ethyloxy, n-propylamino or diethylamino), R^{3A} is not 4-cyclohexylphenyl, when R^{1A} is hydroxymethyl or -C(=O)R^{9C} (wherein R^{9C} represents methoxy, amino, mono-lower alkylamino or di-lower alkylamino) and R^{2A} is carboxyethyl or methoxycarbonylethyl, R^{3A} is not 4-(2-fluorophehyl)phenyl nor biphenyl-4-yl, and when R^{1A} is hydroxymethyl or -C(=O)R^{9D} (wherein R^{9D} represents amino or lower alkoxy) and R^{2A} is methyl, R^{3A} is not biphenyl-4-yl],
or a pharmaceutically acceptable salt thereof.

10. The quinoline derivative or the pharmaceutically acceptable salt thereof according to claim 9, wherein R^{3A} is substituted or unsubstituted biphenylyl or substituted or unsubstituted piperazin-1-yl.

11. The quinoline derivative or the pharmaceutically acceptable salt thereof according to claim 9, wherein R^{3A} is substituted or unsubstituted biphenylyl or piperazin-1-yl having substituted or unsubstituted lower alkyl or substituted or unsubstituted aryl as a substituent on the 4-position.

12. The quinoline derivative or the pharmaceutically acceptable salt thereof according to claim 9, wherein R^{3A} is piperazin-1-yl having substituted or unsubstituted aryl as a substituent on the 4-position.

13. The quinoline derivative or the pharmaceutically acceptable salt thereof according to any one of claims 9 to 12, wherein R^{1A} is lower alkyl, hydroxy lower alkyl, -C(=O)R^{9E} (wherein R^{9E} represents lower alkyl or lower alkoxy) or cyano, and R^{2A} is substituted or unsubstituted lower alkyl.

14. The quinoline derivative or the pharmaceutically acceptable salt thereof according to any one of claims 9 to 13, wherein R^{1A} is methyl, hydroxymethyl, acetyl, methoxycarbonyl or cyano.

15. The quinoline derivative or the pharmaceutically acceptable salt thereof according to any one of claims 9 to 14, wherein n is 1, and R⁴ is halogen.

16. A PDE10A inhibitor which comprises the quinoline derivative or the pharmaceutically acceptable salt thereof according to any one of claims 9 to 15 as an active ingredient.

17. An agent for treating and/or preventing a disease caused by enhancing the activity of PDE10A, which comprises the quinoline derivative or the pharmaceutically acceptable salt thereof according to any one of claims 9 to 15 as an active ingredient.

18. An agent for treating and/or preventing dyskinesia, which comprises the quinoline derivative or the pharmaceutically acceptable salt thereof according to any one of claims 9 to 15 as an active ingredient.

19. An antitumor agent which comprises the quinoline derivative or the pharmaceutically acceptable salt thereof according to any one of claims 9 to 15 as an active ingredient.

20. An agent for treating and/or preventing dyskinesia, which comprises a compound having PDE10A inhibitory activity or a pharmaceutically acceptable salt thereof as an active ingredient.

21. A pharmaceutical composition which comprises the quinoline derivative or the pharmaceutically acceptable salt thereof according to any one of claims 9 to 15 as an active ingredient.

22. Use of the quinoline derivative or the pharmaceutically acceptable salt thereof according to any one of claims 1 to 8 for manufacture of a PDE10A inhibitor.

23. Use of the quinoline derivative or the pharmaceutically acceptable salt thereof according to any one of claims 9 to 15 for manufacture of a PDE10A inhibitor.

24. Use of the quinoline derivative or the pharmaceutically acceptable salt thereof according to any one of claims 1 to 8 for manufacture of an agent for treating and/or preventing a disease caused by enhancing the activity of PDE10A.

25. Use of the quinoline derivative or the pharmaceutically acceptable salt thereof according to any one of claims 9 to 15 for manufacture of an agent for treating and/or preventing a disease caused by enhancing the activity of PDE10A function.

26. Use of the quinoline derivative or the pharmaceutically acceptable salt thereof according to any one of claims 9 to 15 for manufacture of an agent for treating and/or preventing dyskinesia.

27. Use of the quinoline derivative or the pharmaceutically acceptable salt thereof according to any one of claims 9 to 15 for manufacture of an antitumor agent.

28. A method for treating a disease caused by enhancing the activity of PDE10A, which comprises administering an effective amount of the quinoline derivative or the pharmaceutically acceptable salt thereof according to any one of claims 1 to 8.

29. A method for treating a disease caused by enhancing the activity of PDE10A, which comprises administering an effective amount of the quinoline derivative or the pharmaceutically acceptable salt thereof according to any one of claims 9 to 15.

30. A method for treating dyskinesia, which comprises administering an effective amount of the quinoline derivative or the pharmaceutically acceptable salt thereof according to one any of claims 9 to 15.

31. A method for treating a malignant tumor, which comprises administering an effective amount of the quinoline derivative or the pharmaceutically acceptable salt thereof according to any one of claims 9 to 15.

32. Use of a compound having PDE10A inhibitory activity or a pharmaceutically acceptable salt thereof for manufacture of an agent for treating and/or preventing dyskinesia.

33. A method for treating dyskinesia, which comprises administering an effective amount of a compound having PDE10A inhibitory activity or a pharmaceutically acceptable salt thereof.
